# EUROPÄISCHE PATENTSCHRIFT

(11) **EP 0 835 238 B1**
(45) Veröffentlichungstag und Bekanntmachung des Hinweises auf die Patenterteilung: **21.02.2001**
(21) Anmeldenummer: 96922799.0
(22) Anmeldetag: 12.06.1996
(51) Int. Cl.: C07C 237/08, C07C 219/08, C07K 5/06, C12N 15/88

(54) **NEUE KATIONISCHE UND POLYKATIONISCHE AMPHIPHILE, DIESE ENTHALTENDE REAGENZIEN UND DEREN VERWENDUNG**
NEW CATIONIC AND POLYCATIONIC AMPHIPHILES, REACTIVES CONTAINING THE SAME AND THEIR USE
NOUVEAUX AMPHIPHILES CATIONIQUES ET POLYCATIONIQUES, REACTIFS LES CONTENANT ET LEUR UTILISATION

(30) Priorität: 14.06.1995 DE 19521412
(43) Veröffentlichungstag der Anmeldung: 15.04.1998
(73) Patentinhaber: Roche Diagnostics GmbH, 68298 Mannheim (DE)
(72) Erfinder: FERNHOLZ, Erhard, D-82362 Weilheim (DE); VON DER ELTZ, Herbert, D-82362 Weilheim (DE); HINZPETER, Matthias, D-81375 München (DE)
(86) Internationale Anmeldenummer: EP9602541
(87) Internationale Veröffentlichungsnummer: WO9700241

(56) Entgegenhaltungen:
- WO-A-94/05624
- DE-A- 2 835 369
- JOURNAL OF THE AMERICAN CHEMICAL SOCIETY, Bd. 73, Nr. 8, 6.August 1951, DC US, Seiten 3921-3922, XP002015346 HERMANN SCHLENK ET AL.: "The Synthesis of C14-Labeled Glycerol"
- CHEMICAL ABSTRACTS, vol. 59, no. 8, 14.Oktober 1963 Columbus, Ohio, US; abstract no. 8586b, H. P. KAUFMANN ET AL. : "Derivatives of aliphatic diesters of dihydroxyacetone" XP002015348 & NAHRUNG, Bd. 1, Nr. 2, 1963, Seiten 95-105,
- PROCEEDINGS OF THE NATIONAL ACADEMY OF SCIENCES OF USA, Bd. 84, November 1987, WASHINGTON US, Seiten 7413-7417, XP002015347 PHILIP L. FELGNER ET AL.: "Lipofection: A highly efficient, lipid-mediated DNA-transfection procedure" in der Anmeldung erwähnt
- BIOCONJUGATE CHEMISTRY, Bd. 5, Nr. 5, 1994, WASHINGTON US, Seiten 382-389, XP000465949 JEAN-PAUL BEHR: "Gene Transfer with Synthetic Cationic Amphiphiles: Prospects for Gene Therapy" in der Anmeldung erwähnt

## Beschreibung

Die Erfindung betrifft neue kationische bzw. polykationische Amphiphile, welche zur Aggregatbildung mit Makromolekülen, insbesondere mit DNA oder RNA befähigt sind, und deren Einschleusung in prokaryotische oder eukaryotische Zellen.

Amphiphile (z.B. Tenside, Detergenzien) spielen allgemein schon seit einiger Zeit eine Rolle im täglichen Leben. Kationische Amphiphile haben dagegen erst durch die Pionierarbeiten von Felgner in letzter Zeit größere Beachtung gefunden, da man mit ihrer Hilfe DNA und RNA in Zellen einschleusen und diese somit transfizieren kann (P.L. Felgner et al., *Proc. Natl. Acad. Sci.,* USA 84, 7413-7417 ( 1987); P. Hawley-Nelson. WO 94/05624 et al., J.P. Behr, EP 0394111).

Die dabei verwendeten Reagenzien sehen im einzelnen sehr verschieden aus. Dies liegt zumeist daran, daß sie empirisch gefunden wurden. Man konnte also keine Verbindung gezielt für die hier zur Rede stehende Anwendung bereitstellen. Zudem läßt sich durch die Tatsache, daß es Verbindungen gibt, die nur im Gemisch mit anderen Reagenzien eine Wirkung zeigen, die ganze Komplexizität der Transfektion erkennen. Ein Überblick der meisten bislang gefundenen Reagenzien gibt Behr *(Bioconjugate Chem., 5,* 382-389 (1994) und darin angegebene Referenzen). Generell gilt jedoch, daß die überwiegende An Zahl der Reagenzien Liposomen bilden können.

Der Mechanismus der Transfektion mittels kationischer Amphiphile ist noch wenig verstanden. Daß die Liposomen durch ihre positive Ladung mit der DNA einen noch positiv geladenen Komplex bilden und dieser sich an die negativ polarisierte Zellmembran anlagert, erscheint noch plausibel (P.L. Felgner, *Nature 337*, 387-388 (1989)). Wie die Durchdringung der Zellmembran und der Transport zum Zellkern geschieht, ist jedoch ungewiß.

Trotzdem lassen sich für neue Reagenzien neben der Transfektionseffizienz noch eine Reihe anderer Anforderungen formulieren:
- für die Effizienz sollten keine Vorbehandlungen der Zellen wie z.B. Permeabilisierung der Zellmembran durch DMSO. einem Detergenz wie z.B. Digitonin oder Ankratzen vonnöten sein.
- die Reagenzien sollten möglichst keine Toxizität zeigen, insbesondere nicht bei der effektivsten Konzentration und vorzugsweise biologisch abbaubar sein.
- sie sollten auf alle in Frage kommenden Zellen gleichermaßen verwendet werden können.
- sie sollten auf alle in Frage kommenden Zellen gleichermaßen verwendet werden können.
- sie sollten keine Spezifität gegenüber bestimmten DNA-Molekülen haben.
- sie sollten auch in vivo anwendbar sein. Dies bedeutet, daß neben der Toxizität auch die Verträglichkeit mit Serum eine bedeutende Rolle spielt. Gerade hier hat sich häufig eine drastische Abnahme der Transfektionseffizienz gezeigt.

Der Erfindung lag somit die Aufgabe zugrunde, entsprechende Reagenzien, die diesen Anforderungen gleichermaßen genügen, zur Verfügung zu stellen.

Die Aufgabe wird durch bestimmte kationische, lipidische Amphiphile gelöst, mit deren Hilfe sich anionische Verbindungen, wie z.B. DNA in Zellen einschleusen lassen, und die dabei überraschend verbesserte Eigenschaften im Hinblick der oben aufgeführten Anforderungen zeigen.

Insbesondere dienen als Amphiphile Verbindungen der allgemeinen Formel (I) 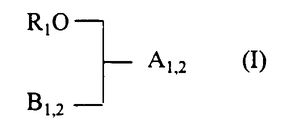 wobei
- R₁: eine gesättigte oder ungesättigte C(O)-C₁₋₂₃ oder gesättigte oder ungesättigte C₁₋₂₄-Gruppe darstellt,
- A₁: eine O-R₂-Gruppe, wobei R₂ die für R₁ angegebene Bedeutung hat und gleich oder von R₁ verschieden sein kann.
- A₂: einen NR₃X- oder einen N⁺R₃R₄R₅Y⁻-Rest, wobei
R₃, R₄, die untereinander gleich oder verschieden sein können, Wasserstoff. eine Alkylgruppe mit 1 bis 4 C-Atomen, eine (CH₂)ₙ -OH- oder eine (CH₂)ₙ-NH₂-Gruppe mit n = 2-6 bedeuten,
R₅, welcher gleich oder verschieden von R₃ oder R₄ sein kann, Wasserstoff, eine Alkylgruppe mit 1 bis 4 C-Atomen, eine (CH₂)ₙ -OH-, eine (CH₂)ₙ-Halogenid-, oder eine ((CH₂)ₘNH)ₒ-(CH₂)ₙ -NH₂-Gruppe bedeuten, wobei m eine ganze Zahl von 2 bis 6 ist und gleich oder verschieden von n oder o sein kann, wobei n eine Zahl von 2 bis 6 und o eine ganze Zahl von 0 bis 4 sein kann,
X zusätzlich zu der Bedeutung für R₅, folgende Bedeutung haben kann: eine amidisch gebundene Aminosäure. ein amidisch gebundenes Peptid bzw. Polypeptid, eine -C(O)-CHR₆N(R₇)₂-, eine -C(O)-CHR₆N⁺(R₇)₃-, eine C(O)-CHR₆N⁺(R₇)₂R₈- oder eine -C(O)-CHR₆N R₇R₈-Gruppe mit der Maßgabe, daß wenn R₃ Wasserstoff ist, X eine amidisch gebundene Aminosäure, ein Aminosäurederivat ein Peptid bzw. Polypeptid bedeutet, wobei
R₆ ein -(CH₂)ₘ-NR₇R₈-, ein -(CH₂)ₘ-N⁺(R₇)₂R₈ oder ein -(CH₂)ₘ -N⁺(R₇)₃-Rest und m eine Zahl von 1 bis 5 sein kann.
R₇ Wasserstoff oder eine Alkylgruppe mit 1 bis 4 C-Atomen,
R₈ eine -(CH₂)ₙ -N(R₇)₂- oder -(CH₂)ₙ -N⁺(R₇)₃ -Gruppe. wobei n eine Zahl von 2 bis 4 ist und R₇ die oben angegebene Bedeutung haben kann und
Y ein pharmazeutisch akzeptables Anion ist.
- B₁: ein NH[C(O)-(CH₂)p -NH]q - Z-Rest, wobei p eine Zahl von 1 bis 6 und q eine Zahl von 0 bis 2 ist.
Z eine amidisch gebundene Aminosäure, ein amidisch gebundenes Peptid bzw. Polypeptid eine -C(O)-CHR₆N (R₇)₂-, eine -C(O)-CHR₆N⁺(R₇)₃-, eine C(O)-CHR₆N⁺(R₇)₂ R₈- oder eine -C(O)-CHR₆NR₇R₈-Gruppe darstellen und R₆ bis R₈ und m die oben angegebenen Bedeutungen haben. und
- B₂: die für A₁ angegebene Bedeutung hat, mit der Maßgabe, daß die Bedeutung von A₁ nur mit B₁, die von A₂ nur mit B₂ gilt.

Bevorzugt sind dabei solche Verbindungen, bei denen die Reste R₁ und A₁ Alkylreste mit 10 bis 20 C-Atomen aufweisen. Darüber hinaus sind solche Verbindungen, in denen A₂ die Bedeutung NR₃X aufweist, bevorzugt, und zwar insbesondere solche. wobei R₃ Wasserstoff und X eine amidisch gebundene Aminosäure, ein geeignetes Aminosäurederivat, ein Peptid bzw. Polypeptid bedeuten.

Als pharmazeutisch akzeptables Anion. Y, kommen insbesondere Halogenide. Monomethylsulfat. Acetat, Trifluoroacetat und Phosphat in Betracht.

Als besonders geeignet haben sich folgende erfindungsgemäßen Verbindungen erwiesen: 2-(6-Carboxyspermyl)-1,3-dioleoyloxy-propylamid, 2-(N,N,N,N',N',N'-Hexamethylornithyl)-1,3-dioleoyloxy-propylamid. 1-(6-Carboxyspermyl)-2,3-dioleoyloxy-propylamid und/oder 2,3-Dioleoyloxy-N-(N-(spermyl)-glycyl)-aminopropan sowie entsprechende Derivate hiervon. Aber auch 2-(6-Carboxyspermyl)-1,3-dimyristoyloxy-propylamid, 2-(1,1,1,5,5,10,10,14, 14, 14-Decamethyl-6-carboxyspermyl)-1,3-dioleoyloxy-propylamid und/oder 2-(N,N,N,N', N',N'-Hexamethyllysyl)-1,3-dioleoyloxy-propylamid haben sich als geeignet erwiesen.

Ähnliche Verbindungen. wie beispielsweise Amin- und Amidinderivate von Glyzerin und Propandiolen. wie z.B. 2-Aminopropanverbindungen, wurden für andere Verwendungen bereits beschrieben, eine Eignung für biologische Anwendungen wurde jedoch nicht nahegelegt (DE-A-2835369; Woltrom et al. Journal of the Am. Soc. (1951) 73(8), S. 3553; Chem. Abs., 59(8), 1963, Spalte 8586, Abs. 6).

Des weiteren betrifft die vorliegende Anmeldung die Herstellung von Reagenzien mit Transfektionseigenschaft. Dabei können die neuen Reagenzien gleichermaßen als Lösung in einem wasser-mischbaren Lösungsmittel oder als wäßrige, liposomale Formulierung angewendet werden. Bei der Erprobung ihrer Transfektionseffizienz fiel auf, sung in einem wasser-mischbaren Lösungsmittel oder als wäßrige, liposomale Formulierung angewendet werden. Bei der Erprobung ihrer Transfektionseffizienz fiel auf,
- daß sie in Serum-freiem Medium bereits eine meistens bessere Effizienz zeigen als andere kommerziell erhältliche Produkte.
- daß sie in Serum-freiem Medium überraschenderweise ein breiteres Plateau des DNA/Reagenz-Verhältnisses bei ähnlicher Transfektionseffizienz zuließen,
- daß sie überraschenderweise die gleiche oder eine bessere Effizienz in Serumhaltigen Medium zeigten,
- daß sich die Effizienz durch liposomale Formulierung noch steigern ließ,
- daß sich überraschenderweise die Verbindungen als weitaus weniger toxisch erwiesen als Verbindungen mit ähnlicher Transfektionseffizienz im Serum-freien System.

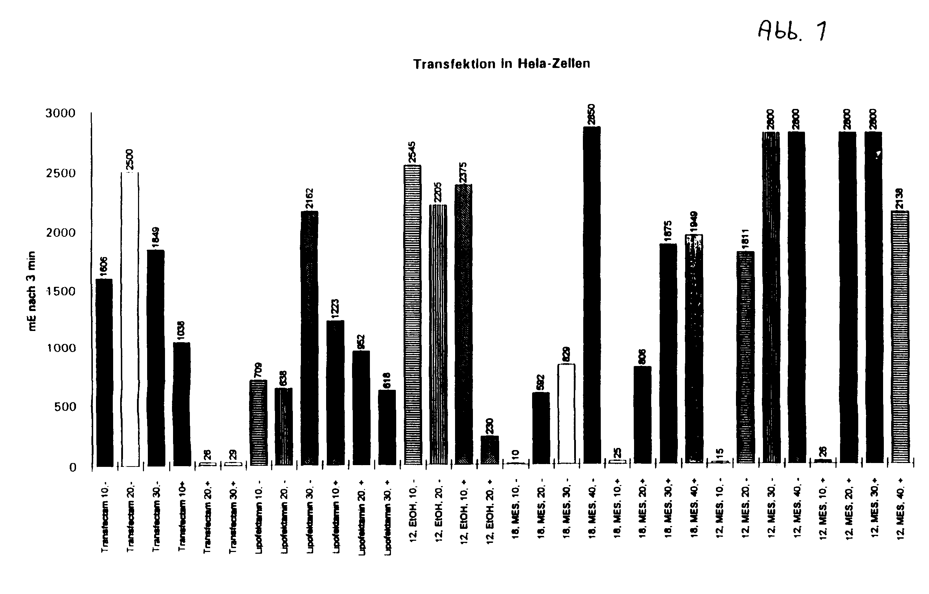

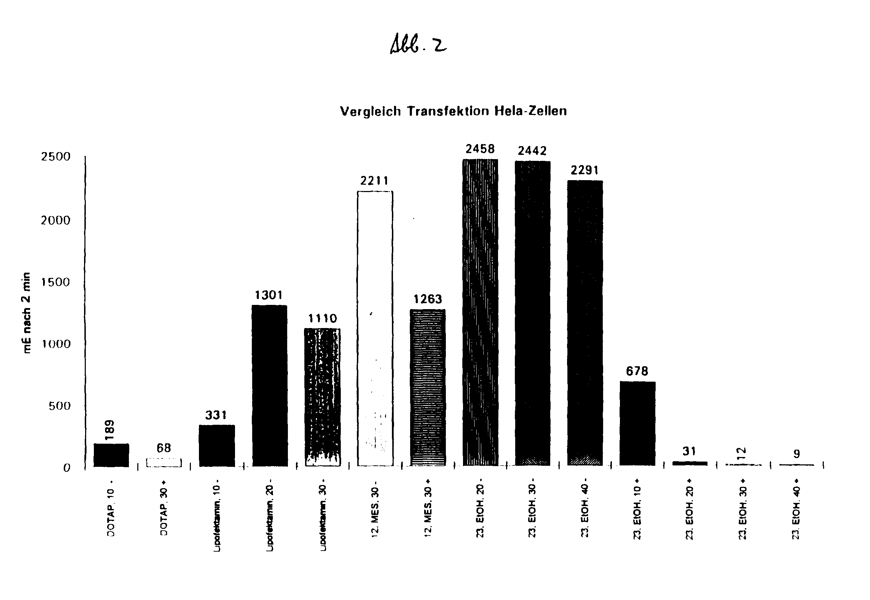

In den Abbildungen 1 und 2 sind die Transfektionseffizienzen einiger ausgewählter Verbindungen im Vergleich zu bekannten Reagenzien dargestellt.

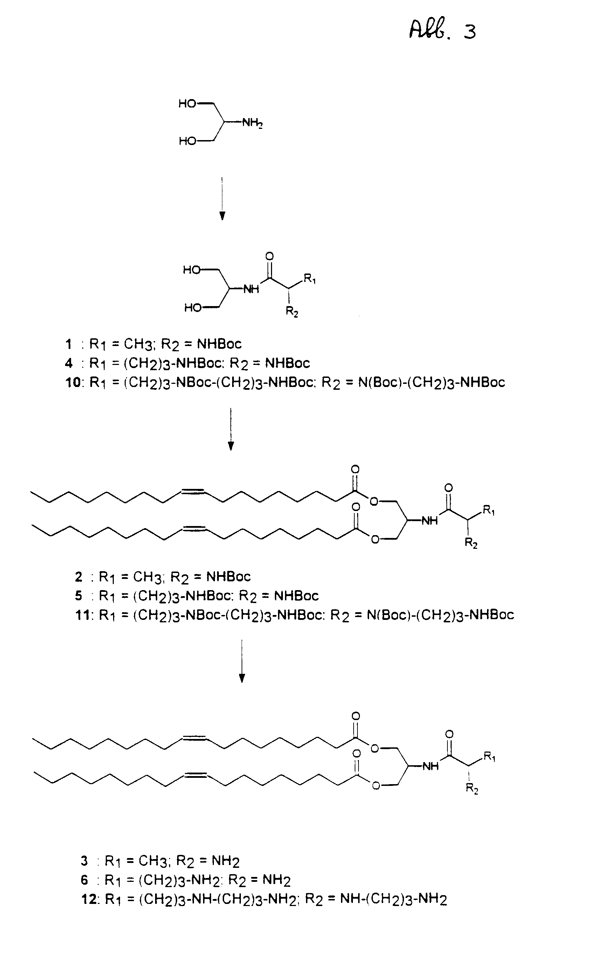

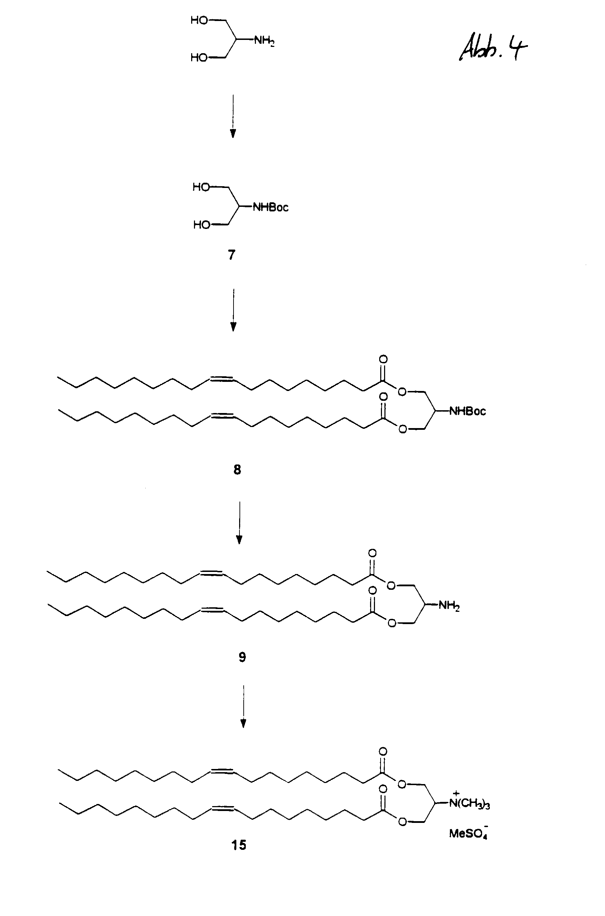

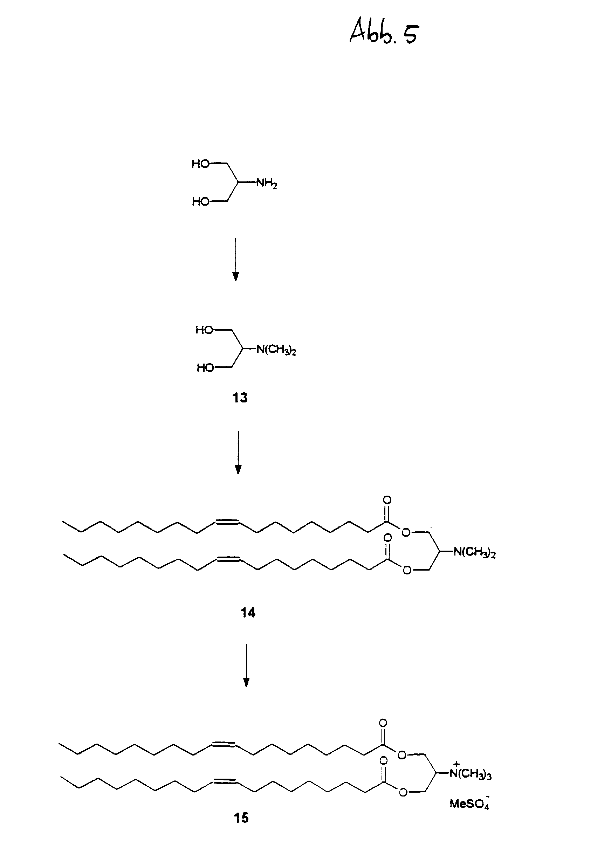

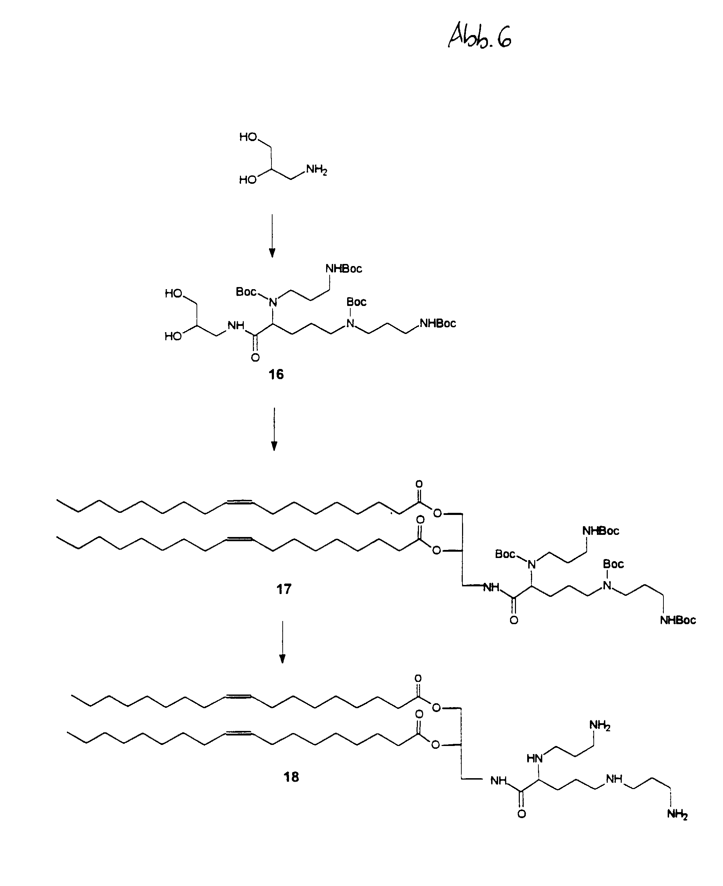

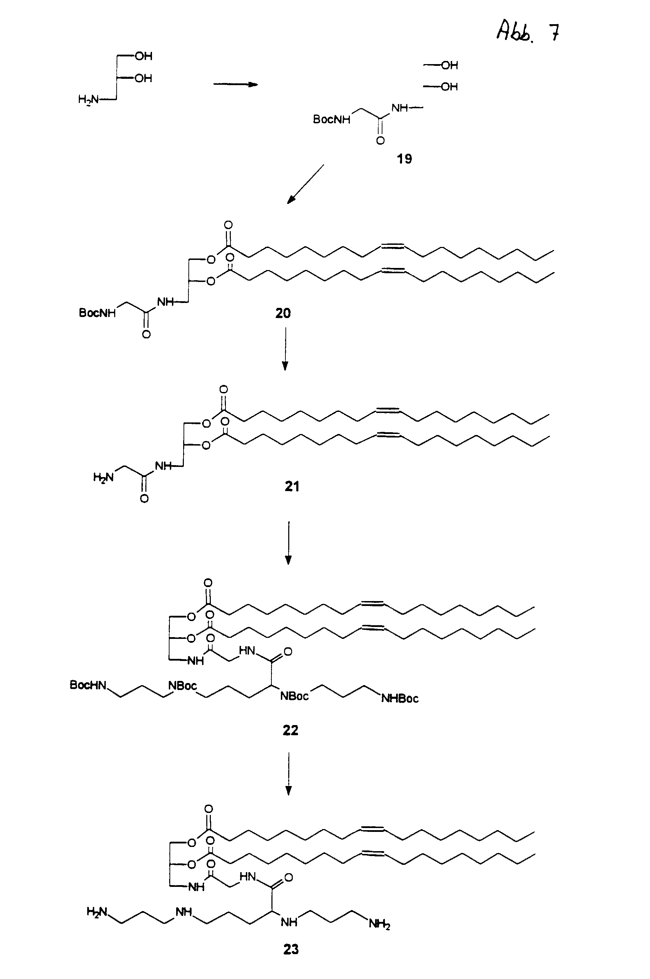

Die Synthese der erfindungsgemäßen Verbindungen erfolgt nach dem für den Fachmann üblichen Methoden (J. March: Advanced Organic Chemistry; John Wiley & Sons, 1985 und M. Bodanszky; Principles of Peptide Synthesis; Springer Verlag, 1984), wobei die Endprodukte ggf. chromatographisch insbesondere durch Ionenaustauscherchromatographie gereinigt werden. Entsprechende Reaktionsschemata sind in den Abbildungen 3 bis 7 angeführt.

Voraussetzung für die Herstellung neuer Transfektionsreagenzen war, daß die entsprechenden Verbindungen kationisch und zur Aggregatbildung geeignet sind, da bislang vor allem solche geladenen Reagenzien zur Transfektion führten (es gibt Beispiele. bei welchen die DNA mittels ungeladener Reagenzien in die Zelle transponiert werden konnte. Hierzu müssen sie dann aber in Liposomen eingeschlossen sein, bei deren Darstellung das zu befördernde Molekül (z.B. DNA) u.U. geschädigt werden kann. Generell wurden durch solche Komplexe keine hohen Effizienzen erzielt (siehe z.B. A. Cudd, C. Nicolau in *Liposome Technology* (Gregoriadis Ed.), (1984) CRC Press Inc. Boca Raton, Fl.; S.F. Alino, M. Bobadilla. M. Garcia-Sanz, M. Lejarreta, F. Unda, E. Hilario in Biochem. Biophys. Res. Commun. 192. 174 (1993)).

Ein weiterer Ansatzpunkt war, daß die Verbindungen dazu imstande sein sollten. Liposomen zu bilden. Es ist dabei nicht klar, warum fast ausschließlich Liposomen-bildende Verbindungen zur Transfektion geeignet sind. Wie neueste Erkenntnisse (H. Gershon. R. Ghirlando. S.B. Guttman, A. Minsky in Biochemistry 32, 7143 (1993) und B. Sternberg, F.L. Sorgi, L. Huang in FEBS Letters 356, 361 (1994) zeigen, tragen sie zur Ausbildung von sog. Meatball-Spaghetti-Strukturen bei. welche zur Transfektion benötigt werden. Ob dies für alle Reagenzien gleichermaßen gilt und in welcher Form diese zum Passieren der Zellmembran beitragen, ist bis heute ungeklärt.

Aus diesen Überlegungen heraus resultierten die folgenden de novo-Synthesen, welche in den Schemata gemäß der Abbildungen 3 bis 7 dargestellt sind.

Für die Bereitstellung von entsprechenden Reagenzien wie beispielsweise pharmazeutischen Formulierungen stehen generell mehrere Methoden gemäß dem Stand der Technik zur Verfügung. Insbesondere werden liposomale und ethanolische Formulierungen verwendet, wobei sich bereits die verschiedenen Liposomen-Arten in ihrer Effizienz unterscheiden können (Zur Darstellung von Liposomen siehe z.B. H. Schreier in Pharmazie in unserer Zeit 11, 97 (1982)).

Die erfindungsgemäßen Verbindungen können jedoch nicht nur als Liposomen, sondern auch in Form anderer Aggregate für die Transfektion verwendet werden. Sowohl bei der Liposomen- wie auch bei der Aggregatbildung können neben den erfindungsgemäßen Verbindungen zusätzlich andere lipidische Verbindungen zugegen sein. Hier sind beispielsweise Verbindungen aus der Klasse der Phospholipide geeignet; aber auch weitere dem Fachmann an sich bekannte Verbindungen können hier zum Einsatz kommen.

Es ist ebenso als überraschend anzusehen, daß sich bei den erfindungsgemäßen, hier erstmals beschriebenen Verbindungen des Typs 3, 6, 12 oder 15 (s. Beispiele 3, 6, 12 und 15) Liposomen erzeugen lassen.

Die Transfektionen wurden exemplarisch an HeLa-Zellen mit pSV2-CAT-Plasmid (Gorman, C.M. et al., Mol. Cell. Biol. 2,1044-1051 (1982)) durchgeführt. Die Transfektionseffizienz wird durch den CAT-ELISA-Kit (Boehringer Mannheim GmbH) bestimmt und ist in den Abbildungen 1 und 2 im Vergleich zu bekannten Reagenzien dargestellt.

Wegen ihrer geringen Toxizität lassen sich diese Verbindungen (selbst oder in Verbindung mit anderen lipidischen Verbindungen) auch in vivo anwenden.

Die folgenden Beispiele erläutern die Erfindung weiter:

### Beispiel 1

### Synthese von 2-(N-tert.-Butyloxycarbonyl-L-alanyl)-1,3-dihydroxy propylamid 1

In einem Rundkolben mit Blasenzähler wurden 756 mg (4 mmol) Boc-L-alanin und 600 µl (4.3 mmol) Triethylamin, gelöst in 10 ml abs. THF, vorgelegt und auf -10 °C abgekühlt. Nun wurden 320 µl (4 mmol) Chlorameisensäuremethylester zugegeben und 30 min bei 0 °C gerührt. Nach Zugabe von 456 mg (5 mmol) 2-Amino-1,3-propandiol wurde soviel Wasser zugesetzt, bis sich eine homogene Lösung bildete und noch 1 h bei Raumtemperatur gerührt. Nach Entfernen des THF wurden 50 ml Ethylacetat zugegeben. Die organische Phase wurde mit ges. NaHCO₃-Lösung und ges. NaCl-Lösung gewaschen und über Na₂SO₄ getrocknet. Nach Entfernung des Lösungsmittels erhielt man 332 mg (32 %) eines farblosen Öls.
^{**1**}**H-NMR (CDCl**_{**3**}**)**: δ = 1.32 (d; 3 H; J=7.1 Hz; CH-CH₃); 1.40 (s; 9 H; C(CH₃)₃); 3.55-3.85 (m; 4 H; CH (CH₂OH)₂); 3.85- 4.0 (m; 1 H; NH-CH); 4.0- 4.5 (m; 3 H; NH-CH, 2 x OH); 5.6-5.8 (m; 1 H; NH-CH (CH₂OH)₂); 7.0-7.35 (m; 1 H; OOC-NH-CH).
^{**13**}**C-NMR (CDCl**_{**3**}**)**: δ = 18.4 (CH-CH₃); 28.2 (C(CH₃)₃), 52.2 (NH-CH-CO); 52.4 (CH(CH₂OH)₂); 61.8 (CH(CH₂OH)₂); 80.2 (C(CH₃); 155.7 (NH-COO); 173.8 (CH-CO-NH).

### Beispiel 2

### Synthese von 2 (N-tert.-Butyloxycarbonyl-L-alanyl)-1,3-dioleoyloxy propylamid 2

In einem Rundkolben wurde eine Lösung von 598 mg (2.28 mmol) 1, 1.610 g (5.70 mmol) Ölsäure 1.176 g (5.70 mmol) DCC und 28 mg (0.23 mmol) Dimethylaminopyridin in 40 ml abs. CH₂Cl₂ über Nacht bei Raumtemperatur gerührt. Anschließend wurde filtriert und das Filtrat einrotiert. Das Rohprodukt wurde durch Säulenchromatographie (Kieselgel, CH₂Cl₂: MeOH = 30:1) gereinigt. Man erhielt 1.450 g, (80 %) eines farblosen Öls.
^{**1**}**H-NMR (CDCl**_{**3**}**)**: δ = 0.87 (t; 6 H; J=6.6 Hz; 2 x CH₂-CH₃); 1.15-1.5 (m; 43 H; CH-CH₃, 2 x CO-(CH₂)₂-(CH₂)₄, 2 x (CH₂)₆-CH₃); 1.43 (s; 9 H; C(CH₃)3; 1.5-1.7 (m; 4 H; 2 x CO-CH₂-CH₂); 1.9-2.1 (m; 8 H; 2 x CH₂-CH=CH-CH₂); 2.30 (t; 4 H; J=7.6 Hz; 2 x CO-CH₂); 4.0-4.25 (m; 5 H; NH-CH, 2 x CH₂OOC); 4.35-4.5 (m; 1 H; NH-CH); 4.9-5.0 (m; 1 H; NH-CH-CH₂); 5.25-5.45 (m; 4 H; 2 x CH=CH); 6.5-6.65 (m; 1 H; OOC-NH-CH).
^{**13**}**C-NMR (CDCl**_{**3**}**)**: δ = 14.0 (2 x C(18)H₃); 22.5 (2 x C(17)H₂); 24.7 (2 x C(3)H₂); 27.0, 27.1, 28.98, 29.04, 29.2, 29.4, 29.56, 29.62 {2 x [C(4)H₂-C(7)H₂, C(12)H₂-c(16)H₂]}; 28.1 (C(CH₃)₃); 31.8 (2 x C(2)H₂); 33.87, 33.88 {2 x [C(8)H₂, C(11)H₂]}, 47.2 (CH(CH₂O₂C-R)₂); 62.4 (CH(CH₂O₂C-R)₂); 129.6, 129.9 (2 x CH=CH); 172.4 (CH-CO-NH); 173.4 (CH₂-COO).

### Beispiel 3

### Synthese von 2-L-Alanyl-1,3-dioleoyloxy-propylamid 3

In einem Rundkolben mit Blasenzähler wurden 702 mg (0.89 mmol) 2, gelöst in 6 ml abs. CH₂Cl₂, vorgelegt und 2 ml (26.12 mmol) TFA zugegeben. Nach 30 min Rühren bei Raumtemperatur wurde die Reaktionsmischung in einem Scheidetrichter überführt und 50 ml CH₂Cl₂ sowie 28 ml 1 *N* NaOH zugegeben. Die organische Phase wurde mit 10 ml ges. NaHCO₃-Lösung gewaschen und über Na₂SO₄ getrocknet. Nach Entfernen des Lösungsmittels erhielt man 599 mg (97 %) eines fast farblosen Öls.
^{**1**}**H-NMR (CDCl**_{**3**}**): δ** = 0.75-0.9 (m; 6 H, 2 x CH₂-CH₃); 1.1-1.4 (m; 43 H; CH-CH₃, 2 x CO-(CH₂)₂ (CH₂)₄, 2 x (CH₂)₆-CH₃); 1.4-1.7 (m; 6 H; NH₂, 2 x CO-CH₂-CH₂); 1.85-2.1 (m; 8 H; 2 x CH₂-CH=CH-CH₂); 2.30 (t; 4 H; J=7.5 Hz; 2 x CO-CH₂); 3.35-3.55 (m; 1 H; NH-CH); 4.0-4.25 (m; 4 H; 2 x CH₂OOC); 4.3-4.5 (m; 1 H; NH-CH); 5.2-5.4 (m; 4 H; 2 x CH=CH); 7.5-7.65 (m; 1 H; CO-NH-CH).
^{**13**}**C-NMR (CDCl**_{**3**}**)**: δ = 14.0 (2 x C(18)H₃); 22.5 (2 x C(17)H₂); 24.7 (2 x C(3)H₂); 27.0, 27.1, 28.9, 29.0, 29.2, 29.4, 29.5, 29.6, {2 x [C(4)H₂-C(7)H₂, C(12)H₂-C(16)H₂]} ; 31.7 (2 x C(2)H₂); 33.9 {2 x [C(8)H₂, C(11)H₂]}; 46.7 (CH(CH₂O₂C-R)₂); 62.5 (CH(CH₂O₂C-R)₂); 129.5, 129.8 (2 x CH=CH); 173.3 (CH-CO-NH); 173.4 (CH₂-COO).

### Beispiel 4

### Synthese von 2-(N,N'-Di-tert.-butyloxycarbonyl-L-ornithyl)-1,3-dihydroxy-propylamid 4

In einem Rundkolben mit Blasenzähler wurden 2.000 g (6 mmol) Boc-L-ornithin und 900 µl 6.45 mmol) Triethylamin gelöst in 25 ml abs. THF, vorgelegt und auf -10 °C abgekühlt. Nun wurden 480 µl (6 mmol) Chlorameisensäuremethylester zugegeben und 30 min bei 0 °C gerührt. Nach Zugabe von 684 mg (7.5 mmol) 2-Amino-1,3-propandiol wurde soviel Wasser zugesetzt, bis sich eine homogene Lösung bildete und noch 1 h bei Raumtemperatur gerührt. Nach Entfernen des Lösungsmittels wurden in Ethylacetat aufgenommen, mit ges. NaHCO₃-Lösung und ges. NaCl-Lösung gewaschen und über Na₂ SO₄ getrocknet. Nach Entfernen des Lösungsmittels erhielt man 1.987 g (82 %) 4 als farblose Kristalle.
^{**1**}**H-NMR (CDCl**_{**3**}**)**: δ = 1.42 (s; 9 H; C(CH₃)₃); 1.4-1.95 (m; 4 H; CH₂-CH₂-CH₂-NH); 3.0-3.2 (m; 2 H; CH₂NH); 3.6-3.8 (m; 4 H; CH(CH₂OH)2); 3.9-4.05 (m; 1 H; NH-CH,) 4.05-4.6 (m; 3 H; NH-CH, 2 x OH); 5.2-5.4 (m; 1 H; NH-CH); 5.8-6.05 (m; 1 H; NH-CH); 7.35-7.5 (m; 1 H, NH-CH).
^{**13**}**C-NMR (CDCl**_{**3**}**)**: δ = 26.1 (CH₂-CH₂-CH₂-NH); 28.4 (C(CH₃)₃); 28.5 (C(CH₃)₃); 30.1 (CH₂-CH₂-NH); 40.0 (CH₂-NH); 52.9 (CH(CH₂OH)₂); 54.4 (NH-CH-CO); 61.8 (CH(CH₂OH)₂); 79.3 (C(CH₃)₃); 80.1 (C(CH₃)₃); 156.1 (NH-COO); 156.5 (NH-COO); 173.3 (CH-CO-NH).

### Beispiel 5

### Synthese von 2-(N,N'-Di-tert.-butyloxycarbonyl-L-lornithyl)-1,3-dioleoxy-propylamid 5

In einem Rundkolben wurde eine Lösung von 1.898 g (4.68 mmol) 4, 3.305 g (11.7 mmol) Ölsäure, 2.414 g (11.7 mmol) DCC und 57 mg (0.47 mmol) Dimethylaminopyridin in 50 ml abs. CH₂Cl₂ über Nacht bei Raumtemperatur gerührt. Anschließend wurde filtriert und das Filtrat einrotiert. Das Rohprodukt wurde durch Säulenchromatographie an Kieselgel (CH₂Cl₂: MeOH = 30:1) gereinigt. Man erhielt 1.678 g (38%) des Produktes als farblosen, wachsartigen Feststoff Smp. 52 - 54 °C.
^{**1**}**H-NMR (CDCl**_{**3**}**)**: δ = 0.75-0.9 (m; 6 H, 2 x CH₂-CH₃); 1.05-1.5 (m; 40 H; 2 x CO-(CH₂)₂-(CH₂)₄, 2 x (CH₂)₆-CH₃); 1.42 (s; 18 H; 2 x C(CH₃)₃); 1.45-1.85 (m; 8 H; CH₂-CH₂-CH₂-NH, 2 x CO-CH₂-CH₂); 1.85-2.1 (m; 8 H; 2 x CH₂-CH=CH-CH₂); 2.2-2.35 (m; 4 H; 2 x CO-CH₂); 3.0-3.35 (2 m; 2 H; CH₂-NH); 4.0-4.25 (m; 5 H; NH-CH, 2 x CH₂OOC); 4.35-4.5 (m; 1 H, NH-CH); 4.7-4.8 (m; 1 H; NH-CH); 5.1-5.2 (m; 1 H, NH-CH); 5.2-5.4 (m; 4 H; 2 x CH=CH); 6.65-6.8 (m; 1 H; OOC-NH-CH).
^{**13**}**C-NMR (CDCl**_{**3**}**)**: δ = 14.0 (2 x C(18)H₃); 22.5 (2 x C(17)H₂); 24.6 (2 x C(3)H₂); 26.2, (CH₂-CH₂-CH₂-NH); 27.0, 27.1, 28.97, 29.04, 29.2, 29.4, 29.56, 29.61, {2 x [C(4)H₂-C(7)H₂, C(12)H₂-C(16)H₂] und CH₂-CH₂-NH}; 28.1, 28.3 (2 x C(CH₃)₃); 31.7 (2 x C(2)H₂); 33.85, 33.86 {2 x [C(8)H₂, C(11)H₂]}; 47.2 (CH(CH₂O₂C-R)₂); 62.3 (CH(CH₂O₂C-R)₂); 129.6, 129.8 (2 x CH=CH); 156.3 (NH-COO); 173.30, 173,33 (CH-CO-NH, 2 x CH₂-COO).

### Beispiel 6

### Synthese von 2-L-Ornithyl-1,3-dioleoyloxy-propylamid 6

In einem Rundkolben wurden 421 mg (0.451 mmol) 5, gelöst in 3 ml abs. CH₂Cl₂, vorgelegt und 1 ml (13.06 mmol) TFA zugegeben. Nach 30 min. Rühren bei Raumtemperatur wurde die Reaktionsmischung mit 200 ml CH₂Cl₂ verdünnt, zweimal mit ges. NaHCO₃-Lösung gewaschen und über Na₂SO₄ getrocknet. Nach Entfernen des Lösungsmittels erhielt man 296 mg (89 %) eines fast farblosen Öls.
^{**1**}**H-NMR (CDCl**_{**3**}**)**: δ = 0.86 (t; 6 H; J-6.7 Hz; 2 x CH₂-CH₃); 1.1-1.45 (m; 40 H; 2 x CO-(CH₂)₂-(CH₂)₄, 2 x (CH₂)₆-CH₃); 1.45-1.7 (m; 8 H; CH₂-CH₂-CH₂-NH₂, 2 x CO-CH₂-CH₂); 1.9-2.1 (m; 8 H; CH₂-CH=CH-CH₂); 2.30 (t; 4 H; J = 7.5 Hz; 2 x CO-CH₂); 2.6-3.1 (m; 4 H; 2 x NH₂); 3.15-3.55 (m; 2 H; CH₂-NH₂); 3.55-3.75 (m; 1 H; NH-CH); 4.0-4.25 (m; 4 H; 2 x CH₂OOC); 4.3 - 4.5 (m; 1 H; NH-CH); 5.2-5.4 (m; 4 H; 2 x CH=CH); 7.65-7.75 (m; 1 H; CO-NHCH).
^{**13**}**C-NMR (CDCl**_{**3**}): δ = 14.0 (2 x C(18)H₃); 22.5 (2 x C(17)H₂); 24.7 (2 x C(3)H₂); 26.99, 27.04, 29.1, 29.3, 29.5, 29.6 {2 x [C(4)H₂-C(7)H₂, C(12)H₂-C(16)H₂], CH₂-CH₂-CH₂NH}; 31.7 (2 x C(2)H₂); 33.9 (2 x [C(8)H₂, C(11)H₂]); 46.7 (CH(CH₂O₂C-R)₂); 54.6 (NH₂-CH-CO); 62.5 (CH(CH₂O₂C-R)₂); 129.5, 129.8 (2 x CH=CH); 173.31, 173.6(CH-CO-NH, 2 x CH₂-COO); 174.7 (CH-CO-NH).

### Beispiel 7

### Synthese von N-tert.-Butyloxycarbonyl-1,3-dihydroxy-propylamin 7

In einem Rundkolben wurde eine Lösung von 1.07 ml (5 mmol) Boc₂O, 697 µl (5 mmol) Triethylamin und 456 mg (5 mmol) 2-Amino-1,3-propandiol in 10 ml THF/Wasser (1:1) über Nacht bei Raumtemperatur gerührt. Nach Entfernen des THF am Rotationsverdampfer wurden 50 ml Ethylacetat und 10 ml ges. NaCl-Lösung zugegeben. Die organische Phase wurde mit 10 ml ges. NaCl-Lösung gewaschen und über Na₂SO₄ getrocknet. Nach Entfernung des Lösungsmittels erhielt man 589 mg (62 %) 7 als farblose Kristalle. Smp. 83 - 85 °C.
^{**1**}**H-NMR (CDCl**_{**3**}): δ = 1.44 (s; 9 H; C(CH₃)₃); 3.4-3.85 (m; 7 H; CH(CH₂OH)₂); 5.3-5.45 (m; 1 H; NH-CH).
^{**13**}**C-NMR (CDCl**_{**3**}**)**: δ = 28.4 (C(CH₃)₃); 53.3 (CH(CH₂OH)₂); 62.6 (CH(CH₂OH)₂); 79.9 (C(CH₃)₃); 156.5 (NH-COO).

### Beispiel 8

### Synthese von N-tert.-Butyloxycarbonyl-1,3-dioleoyloxy-propylamin 8

In einem Rundkolben wurde eine Lösung von 478 mg (2.5 mmol) 7, (1.695 g) (6 mmol) Ölsäure, 1.28 g (6 mmol) DCC und 12 mg (0. 1 mmol) Dimethylaminopyridin in 25 ml abs. CH₂Cl₂ über Nacht bei Raumtemperatur gerührt. Anschließend wurde filtriert und das Filtrat einrotiert. Das Rohprodukt wurde durch Säulenchromatographie an Kieselgel (Hexan: Ethylacetat = 5:1) gereinigt. Man erhielt 1.482 g (82 %) des Produktes als farbloses Öl.
^{**1**}**H-NMR (CDCl**_{**3**}**)**: δ = 0.86 (t; 6 H; J=6.6 Hz; 2 x CH₂CH₃); 1.1-1.5 (m; 40 H; 2 x CO-(CH₂)₂)-(CH₂)₄, 2 x (CH₂)₆-CH₃); 1.43 (s; 9 H; C(CH₃); 1.5-1.7 (m; 4 H; 2 x CO-CH₂-CH₂); 1.9-2.1 (m; 8 H; 2 x CH₂-CH=CH-CH₂); 2.30 (t; 4 H; J=7.5 Hz; 2 x CO-CH₂); 3.95-4.25 (m; 5 H; NH-CH, 2 x CH₂OOC); 4.7-4.85 (m; 1 H; OOC-NH-CH); 5.25-5.45 (m; 4 H; 2 x CH=CH).
^{**13**}**C-NMR (CDCl**_{**3**}**)**: δ = 14.0 (2 x C(18)H₃); 22.5 (2 x C(17)H₂); 24.7 (2 x C(3)H₂); 27.0, 27.1, 28.96, 29.02, 29.2, 29.4, 29.55, 29,62 {2 x [C(4)H₂-C(7)H₂, C(12)H₂-C(16)H₂]}; 28.2 (C(CH₃)₃); 31.8 (2 x C(2)H₂); 33.9 {2 x [C(8)H₂, C(11)H₂]}; 48.4 (CH(CH₂O₂C-R)₂); 62.8 (CH(CH₂O₂C-R)₂); 79.8 (C(CH₃)₃); 129.6, 129.8 (2 x CH=CH); 154.9 (NH-COO); 173.3 (CH₂-COO).

### Beispiel 9

### Synthese von 1,3-Dioleoyloxy-2-propylamin 9

In einem Rundkolben wurden 720 mg (1 mmol) 8, gelöst in 3 ml abs. CH₂Cl₂, vorgelegt und 1 ml ( 13.06 mmol) TFA zugegeben. Nach 30 min Rühren wurde die Reaktionsmischung mit 50 ml CH₂Cl₂ verdünnt, mit 10 ml ges. NaHCO₃-Lösung gewaschen und über Na₂SO₄ getrocknet. Nach Entfernen des Lösungsmittels erhielt man 589 m (95 %) eines farblosen Öls.
^{**1**}**H-NMR (CDCl**_{**3**}**)**: δ = 0.87 (t; 6 H; J=6.6 Hz; 2 x CH₂-CH₃); 1.1-1.5 (m; 40 H; 2 x CO-(CH₂)₂)-(CH₂)₄, 2 x (CH₂)₆-CH₃); 1.35-1.5 (m; 2 H; CH-NH₂); 1.5-1.7 (m; 4 H; 2 x CO-CH₂-CH₂); 1.85-2.1 (m; 8 H; 2 x CH₂-CH=CH-CH₂); 2.32 (t; 4 H; J=7.5 Hz; 2 x CO-CH₂); 3.2-3.35 (m; 1 H; CH-NH₂); 3.95-4.15 (m; 4 H; 2 x CH₂OOC); 5.25-5.45 (m; 4 H; 2 x CH=CH).
^{**13**}**C-NMR (CDCl**_{**3**}**)**: δ = 14.0 (2 x C(18)H₃); 22.5 (2 x C(17)H₂); 24.8 (2 x C(3)H₂); 27.0, 27.1, 28.96, 28.99, 29.03, 29.2, 29.4, 29.55, 29.63 {2 x [C(4)H₂-C(7)H₂, C(12)H₂-C(16)H₂]}; 31.8 (2 x C(2)H₂); 34.0 {2 x [C(8)H₂, C(11)H₂]}; 49 {CH(CH₂O₂C-R)₂}; 65.7 {CH(CH₂O₂C-R)₂}; 129.6, 129.9 (2 x CH=CH); 173.4 (CH₂-COO).

### Beispiel 10

### Synthese von 2-(N,N',N",N"'-Tetra-tert.-butyloxycarbonyl-6-carboxy-spermyl)-1,3-dihydroxy-propylamid 10

Zu einer Lösung von 202 mg (312 µmol) Tetra-Boc-spermin und 47 µl (335 µmol) Triethylamin in 2 ml abs. THF wurden bei - 10 °C 25 µl (312 µmol) Chlorameisensäuremethylester zugegeben und 30 min bei 0 °C gerührt.

Nach anschließender Zugabe von 36 mg 390 µmol) 2-Amino-1,3-propandiol wurde soviel Wasser zugesetzt, bis sich eine homogene Lösung bildete und danach noch 1 h bei Raumtemperatur gerührt. Nach Entfernen des Lösungsmittels am Rotationsverdampfer wurden ca. 30 ml Ethylacetat zugegeben. Die organische Phase wurde mit ges. NaHCO₃-Lösung und ges. NaCl-Lösung gewaschen und über Na₂S0₄ getrocknet. Nach Entfernen des Lösungsmittels erhielt man 208 mg (93 %) eines farblosen, schaumigen Feststoffs.
^{**1**}**H-NMR (CDCl**_{**3**}): δ = 1.3-1.47 (m; 36 H; 4 x C(CH₃)₃); 1.5-2.05 (m; 8 H, 2 x N-CH₂-CH₂-CH₂-N, N-CH-CH₂-CH₂-CH₂-N); 2.85-3.4 (m; 10 H; 5 x N-CH₂); 3.65-3.8 (m; 4 H; 2 x CH₂-OH); 3.8-4.5 (m; 4 H; 2 x -CH-, 2 x CH₂-OH); 4.8-5.1 (s(breit); 1 H; CH₂-NH); 5.25-5.4 (s(breit); 1 H; CH₂-NH); 7.05-7.15 (s(breit); 1 H, NH-CO).
^{**13**}**C-NMR (CDCl**_{**3**}**)**: δ = 25.0, 29.5 (N-CH-CH₂-CH₂-CH₂-N); 28.0, 28.2, 28.3 (4 x C(CH₃)₃); 38.0, 38.1 (2 x N-CH₂-CH₂-CH₂-N); 42.9, 45.0, 47.0 (übrige Spermyl-CH₂-Kohlenstoffe); 52.4 (CH(CH₂-OH)₂); 59.8 (CH-CO); 63.1 (CH(CH₂-OH)₂); 79.3, 79.5, 81.1 (4 x CO-O-C)CH₃)₃); 156.0, 156.1 (4 xCO-O-C(CH₃)₃); 171.1 (CO-NH).

### Beispiel 11

### Synthese von 2-(N,N',N",N'''-Tetra-tert,-butyloxycarbonyl-6-carboxy-spermyl)-1,3-dioleoyloxy-propylamid 11

In einem geschlossenen Rundkolben wurde eine Lösung von 208 mg (289 µmol) **10**, 204 mg (72 µmol) Ölsäure, 149 mg (723 µmol) DCC und 4 mg (29 µmol) Dimethylaminopyridin in 6 ml abs. CH₂Cl₂ über Nacht bei Raumtemperatur gerührt.

Danach wurde filtriert, mit Hexan/Ether (3:1) nachgewaschen und das Filtrat einrotiert. Nach Säulenchromatographie an Kieselgel (CH₂Cl₂/MeOH = 20:1) erhielt man 160 mg (44 %) eines farblosen Öls.
^{**1**}**H-NMR (CDCl**_{**3**}**)**: δ = 0.8-0.95 (m; 6 H; 2 x CH₂-CH₃); 1.2-1.4 (m; 40 H; 2 x CO-(CH₂)₂-(CH₂)₄, 2 x (CH₂)₆ CH₃); 1.4-1.55 (m; 36 H; 4 x C(CH₃)₃); 1.55-1.75 (m; 10 H; 3 x (-CH₂-CH₂-CH₂-)ₛₚₑᵣₘ, 2 x CO-CH₂-CH₂-); 1.85-2.05 (m; 10 H; (-CH-CH₂-CH₂-)ₛₚₑᵣₘ, 2 x CH₂-CH=CH-CH₂); 2.25-2.35 (m; 4 H; 2 x CO-CH₂); 3.0-3.35 (m; 10 H; 5 x N-CH₂); 4.0-4.4 (m; 6 H; 2 x CH-N, (CH(CH₂O₂C-R)₂); 4.5-5.1 (s(sehr breit); 1 H; NH-CH₂); 5.25-5.35 (m; 4 H; 2 x CH=CH); 6.8-7.0 (s(breit); 1 H; NH-CO).
^{**13**}**C-NMR (CDCl**_{**3**}): δ = 14.4 (2 x C(18)H₃); 23.0 (2 x C(17)H₂); 25.2 (2 x C(3)H₂); 27.6 (C(8)H₂, C(11)H₂; 29.5, 29.6, 29.7, 29.9, 30.1, 32.3 ((CH-CH₂-CH₂-CH₂-)ₛₚₑᵣₘ, 2 x N-CH₂-CH₂-CH₂-N, 2 x C(4)H₂-C(7)H₂, 2 x C(12)H₂-C(16)H₂); 28.7, 28.8 (4 x C(CH₃)₃); 34.0 (2 x C(2)H₂); 38.5, 44.6, 46.8, 47.6 (2 x N-CH₂-CH₂-CH₂-N, (CH-CH₂-CH₂-CH₂-)ₛₚₑᵣₘ, CH(CH₂O₂C-R)₂); 59.5 (CO-CH); 62.8 (CH(CH₂O₂C-R)₂); 79.5, 80.1, 81.5 (4 x CO-O-C(CH₃)₃); 130.07, 130.37 (2x CH=CH); 156.3 (4 x CO-O-C(CH₃)₃); 173.7 (CO-NH, 2 x CH₂COO).

### Beispiel 12

### Synthese von 2-(6-Carboxy-spermyl)-1,3-dioleoyloxy-propylamid 12

In einem Rundkolben mit Blasenzähler wurden 85 mg (68 µmol) **11** in 1 ml abs. CH₂Cl₂ gelöst und mit 400 µl TFA versetzt. Die Reaktionsmischung wurde 45 min lang bei Raumtemperatur gerührt. Anschließend wurde mit ca. 20 ml CH₂Cl₂ versetzt, mit ges. NaHCO₃-Lösung gewaschen über Na₂SO₄ getrocknet. Nach Entfernen des Lösungsmittels erhielt man 56 mg (97 %) eines farblosen Öls.
^{**1**}**H-NMR (CDCl**_{**3**}**)**: δ = 0.8-0.95 (m; 6 H; 2 x CH₂-CH₃); 1.15-1.4 (m; 40 H; 2 x CO-(CH₂)₂-(CH₂)₄, 2 x (CH₂)₆-CH₃); 1.5-1.9 (m; 12 H; 3 x (-CH2-CH₂-CH₂-)ₛₚₑᵣₘ, 2 x CO-CH₂-CH₂-, (-CH-CH₂-CH₂-)ₛₚₑᵣₘ); 1.9-2.1 (m; 8 H; 2 x CH₂-CH=CH-CH₂); 2.2- 2.35 (m; 4 H; 2 x CO-CH₂); 2.45-3.3 (m; 10 H; 5 x N-CH₂); 4.0-4.59 (m; 12 H; 2 x CH-N, (CH(CH₂O₂C-R)₂, 2 x NH₂, 2 x NHₛₚₑᵣₘ); 5.25-5.45 (m; 4 H; 2 x CH=CH; 7.6-7.9 (s(breit); 1 H; NH-CO).
^{**13**}**C-NMR (CDCl**_{**3**}**)**: δ = 14.0 (2 x C(18)H₃); 22.5 (2 x C(17)H₂); 24.7 (2 x C(3)H₂); 27.0, 27.1 (C(8)H₂, C(11)H₂); 29.0, 29.1, 29.2, 29.4, 29.60, 29.61, 31.8 ((CH-CH₂-CH₂-CH₂-)ₛₚₑᵣₘ, 2 x N-CH₂-CH₂CH₂-N, 2 x C(4)H₂-C(7)H₂, 2 x C(12)H₂-C(16)H₂); 33.9 (2 x C(2)H₂); 41.0, 47.3, 48.8 (2 x N-CH₂-CH₂-CH₂-N, (CH-CH₂-CH₂-CH₂-)ₛₚₑᵣₘ, CH(CH₂O₂C-R)₂); 62.0, 62.9 (CO-CH, CH(CH₂O₂C-R)₂); 129.5, 129.9 (2 x CH=CH); 173.4, 174.2 (CO-NH, 2 x CH₂COO).

### Beispiel 13

### Synthese von 2-Dimethylamino-1,3-propandiol 13

In einem Rundkolben wurden 456 mg (5 mmol) 2-Amino-1,3-propandiol vorgelegt und unter Eiskühlung 1.13 ml (25 mmol) Ameisensäure (85 %, d = 1.20)versetzt. Anschließend wurden 895 µl (12 mmol) Formaldehyd (37 %) zugefügt und die Mischung für 10 h im Wasserbad auf 80 °C erhitzt. Nach dem Abkühlen wurden 3 ml 2 N Salzsäure zugesetzt. Die Isolation des Produktes erfolgte durch Chromatographie über 25 g sauren Ionenaustauscher (DOWEX). Das so erhaltene Rohprodukt wurde durch Kurzwegdestillation im Ölpumpenvakuum gereinigt. Man erhielt 253 mg (42 %) eines farblosen Öls.
Sdp. _{lmbar} = 105 °C
^{**1**}**H-NMR (DMSO-d**_{**6**}**)**: δ = 2.25 (s; 6 H; N(CH₃)₂); 2.31-2.45 (m; 1 H; CH(CH₂OH)₂); 3.35-3.55 (m; 4 H; CH(CH₂OH)₂); 4.24 (s(breit); 2 H; CH(CH₂OH)₂).
^{**13**}**C-NMR (DMSO-d**_{**6**}**)**: δ = 41.8 (N(CH₃)₂); 58.7 (CH(CH₂OH)₂); 66.7 (CHCH₂OH)₂).

### Beispiel 14

### Synthese von 2-Dimethylamino-(1,3-dioleoyloxy)-propan 14

Eine Lösung von 183 mg (1.54 mmol) 13, 1.088 g (3.85 mmol) Ölsäure, 795 mg (3.85 mmol) DCC und 18 mg (154 µmol) Dimethylaminopyridin in 20 ml abs. CH₂Cl₂ wurde über Nacht bei Raumtemperatur gerührt. Danach wurde filtriert, mit Hexan/Ether (3:1) nachgewaschen und das Filtrat einrotiert. Nach Säulenchromatographie an Kieselgel, (CH₂Cl₂/MeOH 30:1 ) erhielt man 444 mg (45 %) des Produkts als farbloses Öl.
^{**1**}**H-NMR (CDCl**_{**3**}): δ = 0.85-0.95 (m; 6 H; 2 x CH₂-CH₃); 1.2-1.4 (m; 40 H; 2 x CO-(CH₂)₂)-(CH₂)₄, 2 x (CH₂)₆-CH₃); 1.55-1.7 (m; 4 H; 2 x CO-CH₂-CH₂-); 1.95-2.05 (m; 8 H; 2 x CH₂-CH=CH-CH₂); 2.3-2.35 (m; 4 H; 2 x CO-CH₂-CH₂-); 2.39 (s: 6 H, N(CH₃)₂); 2.9-3.0 (m; 1 H; CH(CH₂O₂C-R)₂); 4.1-4.3 (m; 4 H, CH(CH₂O₂C-R)₂); 5.3-5.4 (m, 4 H, 2 x CH=CH).
^{**13**}**C-NMR (CDCl**_{**3**}): δ = 14.4 (2 x C(18)H₃); 23.0 (2 x C(17)H₂); 25.3 (2 x C(3)H₂); 27.55, 27.6 (C(8)H₂, C(11)H₂); 29.50, 29.53, 29.67, 29.7, 29.8, 29.9, 30.06, 30.14. 32.3 (2 x C(4)H₂-C(7)H₂, 2 x C(12)H₂-C(16)H₂); 34.7 (2 x C(2)H₂); 42.4 (N(CH₃)₂); 61.6, 61.9 (CH(CH₂O₂C-R)₂); 130.09, 130.36 (2 x CH=CH); 173.9 (2 x CH₂COO).

### Beispiel 15

### Synthese von N-[2-(1,3-Dioleoyloxy)propyl]-N,N,N-trimethylammoniummethylsulfat 15

Zu einer Lösung von 200 mg (309 µmol) **14** in 1.55 ml Ethylacetat/Hexan (1:1) wurden bei 0 °C 66 µl (696 µmol) Dimethylsulfat zugefügt und die Reaktionsmischung für 24 h bei 4 °C gerührt. Anschließend wurde das Lösungsmittel entfernt. Man erhielt 213 mg (89 %) eines farblosen Öls.
^{**1**}**H-NMR (CDCl**_{**3**}**)**: δ = 0.75-0.9 (m; 6 H; 2 x CH₂-CH₃); 1.05-1.4 (m; 40 H; 2 x CO-(CH₂)₂)-(CH₂)₄, 2 x (CH₂)₆-CH₃); 1.4-1.65 (m; 4 H; 2 x CO-CH₂-CH₂-); 1.8-2.1 (m; 8 H; 2 x CH₂-CH=CHCH₂); 2.25-2.4 (m; 4 H; 2 x CO-CH₂-CH₂-); 3.3-3.45 (s(breit); 9 H; N(CH₃)₃); 3.61 (s; 3 H; CH₃-O-S); 4.1-4.2 (m; 1 H; CH(CH₂O₂C-R)₂); 4.4-4.65 (m; 4 H; CH(CH₂O₂C-R)₂); 5.2-5.4 (m; 4 H; 2 x CH=CH).
^{**13**}**C-NMR (CDCl**_{**3**}): δ = 13.8 (2 x C(18)H₃); 22.4 (2 x C(17)H₂); 24.4 (2 x C(3)H₂); 26.9, 27.0 (C(8)H₂, C(11)H₂); 28.8, 28.9, 29.0, 29.05, 29.3, 29.46, 29.5, 31.6 (2 x C(4)H₂-C(7)H₂, 2 x C(12)H₂-C(16)H₂); 33.6 (2 x C(2)H₂); 53.0, 54.0 (N(CH₃)₃, CH₃-O-S); 58.7 (CH(CH₂O₂C-R)₂); 69.8 (CH(CH₂O₂C-R)₂); 129.4, 129.8 (2 x CH=CH); 172.3 (2 x CH₂COO).

### Beispiel 16

### N,N',N",N"'-Tetra-tert.-butyloxycarbonyl-6-carboxy-spermyl-2,3-dihydroxy-1-propylamid 16

Eine Lösung von 300 mg (0.47 mmol) Tetra-BOC-carboxyspermin in 4 ml Dichlormethan wurde nacheinander mit 59 mg (0.51 mMol) Hydroxysuccinimid gelöst in 2 ml Dichlormethan/THF (1:1) und 106 mg (0.51 mMol) Dicyclohexyl-carbodiimid gelöst in 2.2 ml Dichlormethan ersetzt und 4 Tage bei Raumtemperatur gerührt. Nach Entfernen des Lösungsmittels wurde der Rückstand in Essigsäureethylester aufgenommen, filtriert und aus dem klaren Filtrat das Lösungsmittel entfernt. Der Rückstand (335 mg (0.45 mMol) Tetra-BOC-Carboxyspermin-hydroxy-succinimidylester) wurde in 2.18 ml Dimethylformamid aufgenommen und mit 41 mg (0.45 mMol) (±)-1-Amino-2,3-propandiol und 61.5 µl (0.45 mMol) Triethylamin versetzt. Nach 3tägigem Rühren entfernte man das Lösungsmittel und verteilte den Rückstand zwischen Ether und Wasser. Die organische Phase wird getrocknet. Nach Entfernen des Lösungsmittels erhielt man ein öliges Produkt, das direkt weiterverwendet werden kann.

### Beispiel 17

### N,N',N",N"'-Tetra-tert.-butyloxycarbonyl-6-carboxy-spermyl-2.3-dioleoyloxy-1-propylamid 17

Eine Lösung von 252 mg (0.35 mMol) **16** in 1.4 ml Tetrachlorkohlenstoff wurde mit 218 mg (0.77 mMol) Ölsäure, 180 mg (0.87 mMol) Dicylohexylcarbodiimid und einer katalytischen Menge Dimethylaminopyridin versetzt. Es wurde über Nacht gerührt. Nach Entfernung des Lösungsmittels wurde der Rückstand chromatographiert (Kieselgel, Dichlormethan mit bis zu 10 % ansteigendem Methanolanteil). Man erhielt 392 mg (90 % d. Th.) eines Öls.
^{**13**}**C-NMR:** δ = 173.8, 173.3 (Ölsre.-C=O), 156 (C(=O)-N, 130.0, 129.7 (Ölsre. C=C), 80.97, 79.67, 78.94 (quart.-BOC), 70.3 (Glyc. sn2), 62.6 (Glyc. snl), 59.1 (Sperm.-), 46.5, 44.3, 43.7, 43.1 (Sperm. N-CH₂), 39.5 (Glyc. sn3), 38.2 (Sperm. N-CH₂), 34.9 (Ölsre. C-2), 31.9 (Ölsre. C-16), 29.8-29.1 (Ölsre.-CH₂), 28.4 (BOC-CH₃), 27.2 (Ölsre. C-8,11) 25.0 (Ölsre. C-3), 22.7 (Ölsre. C-17), 14.1 (Ölsre. C-18).

### Beispiel 18

### (6-Carboxy-spermyl)-2,3-dioleoyloxy-1-propylamid 18

Eine Lösung von 195 mg (0. 16 mmol) **17** in 5 ml Dichlormethan wurde bei Raumtemperatur mit 5 ml TFA versetzt. Nach 20 min. Rühren wurde mit 100 ml Dichlormethan verdünnt und mit ges. Natriumhydrogencarbonat-Lösung gewaschen. Die vereinigten organischen Phasen wurden getrocknet und das Lösungsmittel abgedampft. Man erhielt 134 mg (99 %) eines zähen Öls.
^{**1**}**H-NMR** (500 MHz, CDCl₃/CD₃COOD 5:1) δ = 5.33 (Ölsre. 9, 10-H), 5.16 (Glycerin 2-H), 4.25 und 4.06 (Glycerin 1-H), 4.15 (Spermin- -H), 3.55 und 3.33 (Glycerin 3-H), 3.18 und 3.05 (Spermin N-CH₂), 2.31 (Ölsre. 2-H), 2.21 (Spermin -CH₂), 2.01 (Ölsre. 8, 11-H), 1.98 (Spermin- ), 1.83 (Spermin CH₂), 1.57 (Ölsre. 3-H), 1.30 (Ölsre. 4-7, 12-17-H), 0.87 ppm (Ölsre. 18-H).
^{**13**}**C-NMR**: δ = 173.82 und 173.53 (Ölsre. C-1), 154.06 (C(=O)-N), 130.17 u. 129.81 (Ölsre. C-9 u. C-10), 70 (Glycerin C-2), 63.2 (Glycerin C-1), 39.8 (Glycerin C-3), 34.25, 34.05, 33.97 (Ölsre. C-2), 32.70, 31.93 (Ölsre. C-16), 30.82; 29.79, 29.57 u. 29.35 (Ölsre. CH₂), 27.25 (Ölsre. C-8, 11) 25.64, 25.53, 25.32, 24.97 (Ölsre. C-3), 24.71, 22.70 (Ölsre. C-17), 14.13 ppm (Ölsre. C-18).
MS (FAB): MH⁺: 848.6 (Ber. 848.8).

### Beispiel 19

### Synthese von N-((N-Boc)-Glycyl)-aminopropan-2,3-diol 19

10.00 g (36.73 mmol) Boc-Gly-OSu (Bachem) wurden zusammen mit 3.35 g (36.77 mmol) 1-Amino-2,3-propandiol in 50 ml DMF gelöst und 18 h gerührt. Das Produkt wurde durch Flashchromatographie (CH₂,Cl₂/MeOH; 95:5 v/v) gereinigt. Man erhielt 8.94 g (98 %) **19** als farbloses Öl.
^{**1**}**H-NMR (CDCl**_{**3**}**)**: δ = 1.45 (s; 9 H; CH₃); 3.28 (m; 1 H; CH₂-OH); 3.39 (m: 1 H; CH₂-OH); 3.35 (m; 1 H; CH₂-N_{Aminoprop.}); 3.38 (m; 1 H; CH₂-N_{Aminoprop.}); 3.78 (s; 3 H; CH-OH; CH₂-NH_{Gly}); 4.41 (br s; 1 H; OH); 4.67 (br s; 1 H; OH); 6.04 (tr; 1 H; NH); 7.45 (tr; 1 H; NH).
^{**13**}**C-NMR (CDCl**_{**3**}**)**: δ = 28.4 (CH₃); 42.2 (CH₂-N_{Gly}); 44.0 (CH2-N_{Amminoprop.}); 64.0 (CH₂-OH); 70.8 (CH-OH); 80.3 (quart. C_{Boc}); 156.6 (NCO₂); 174.5 (NCO).

### Beispiel 20

### Synthese von 2,3-Dioleoyloxy-N-((N-Boc)-glycyl)-aminopropan 20

8.90 g (35.85 mmol) **19** wurden zusammen mit 22.70 g (110.02 mmol) DCC und 26.60 g (94.17 mmol) Ölsäure in 50 ml DMF gelöst. Dazu wurden 0.42 g (3.44 mmol) DMAP gegeben und 2 d gerührt. Danach wurde abfiltriert, einrotiert und das Produkt chromatographisch (CH₂,Cl₂/MeOH; 99:1 v/v) gereinigt. Ausbeute: 19.25 g **20** als farbloses Öl (69 %).
^{**1**}**H-NMR (CDCl**_{**3**}**)**: δ = 0.89 (tr; 6 H; CH_{3,Ölsäure}); 1.30 (br s; 40 H; C-CH₂-C); 1.45 (br s; 9 H; CH_{3,Boc}); 1.62 (m; 4 H; CH₂-CH₂C=O); 2.03 (m; 8 H; CH₂-CH=CH); 2.32 (tr; 4 H; CH₂C=O); 3.42-3.57 (m; 2 H; CH₂-N_{Aminoprop.}); 3.75 (d; 2 H; CH₂-N_{Gly}); 4.12 (dd; 1 H; CH₂-O_{Aminoprop.}); 4.27 (dd; 1 H; CH₂-O_{Aminoprop.}); 5.08 (m; 1 H, CH-O); 5.20 (s; 1 H; NH); 5.34 (m; 4 H; CH=CH); 6.58 (tr; 1 H; NH).
^{**13**}**C-NMR (CDCl**_{**3**}**)**: δ = 14.0 (CH_{3,Ölsäure}); 22.7 (CH_{2,Ölsäure}); 24.9 (CH_{2,Ölsäure}); 27.2 (CH_{2,Ölsäure}); 27.2 (CH_{2,Ölsaure}); 28.3 (CH_{2,Ölsäure}); 29.2 (CH_{2,Ölsäure}); 29.3 (CH_{3,Boc}); 29.5 (CH_{2,Ölsäure}); 29.8 (CH_{2,Ölsäure}); 31.9 (CH_{2,Ölsäure}); 34.0 (CH_{2,Ölsäure}); 34.2 (CH₂ _{Ölsaure}); 39.7 (CH₂-N_{Gly}); 44.5 (CH₂-N_{Aminoprop.}); 62.7 (CH₂-O_{Aminoprop.}); 70.2 (CH-O_{Aminoprop.}); 80.3 (quart. C_{Boc}); 129.7 (CH=CH); 129.7 (CH=CH); 130.0 (CH=CH) 130.0 (CH=CH); 156.1 (NCO₂); 169.8 (NCO); 173.2 (CO_{Ölsaure}); 173.4 (CO_{Ölsäure}).

### Beispiel 21

### Synthese von 2,3-Dioleoyloxy-N-(glycyl)-aminopropan 21

15.20 g (19.56 mmol) **20** wurden in 200 ml CH₂Cl₂/TFA (3:1 v/v) aufgenommen und 30 min gerührt. Danach wurde die Lösung mit 200 ml CH₂Cl₂ verdünnt und mit 200 ml ges. NaHCO₃-Lösung ausgeschüttelt. Die organische Phase wurde über MgSO₄ getrocknet und einrotiert. Ausbeute: 11.64 g (88 %) **21** als Öl.
^{**1**}**H-NMR (CDCl**_{**3**}**)**: δ = 0.88 (tr; 6 H; CH₃); 1.29 (br s; 40 H; C-CH₂-C); 1.62 (m; 4 H; CH₂-CH₂C=O); 1.82 (s; 2 H; CH₂-N_{Gly}); 2.01 (m; 8 H; CH₂-CH=CH); 2.32 (tr; tr; 4 H; CH₂C=O); 3.36 (s; 2 H; NH₂); 3.43-3.62 (m; 2 H; CH₂-N_{Aminoprop.}); 4.12 (dd; 1 H; CH₂-O_{Aminoprop.}); 4.28 (dd; 1 H; CH₂-O_{Aminoprop.}); 5.14 (m; 1 H; CH-O_{Aminoprop.}); 5.34 (m; 4 H; CH = CH); 7.60 (tr; 1 H; NH).
^{**13**}**C-NMR (CDCl**_{**3**}**)**: δ = 14.0 (CH₃); 22.6 (CH_{2,Ölsäure}); 24.8 (CH_{2,Ölsäure}); 24.8 (CH₂ _{Ölsäure}); 27.1 (CH_{2,Ölsäure}); 29.0 (CH_{2,Ölsäure}); 29.1 (CH_{2,Ölsäure}); 29.2 (CH_{2,Ölsäure}); 29.4 (CH_{2,Ölsäure}); 29.6 (CH_{2,Ölsäure}); 31.8 (CH_{2,Ölsäure}); 34.0 (CH_{2,Ölsäure}); 34.2 (CH_{2,Ölsäure}); 39.1 (CH₂-N_{Gly}); 44.5 (CH₂-N_{Aminoprop.}); 62.7 (CH₂-N_{Aminoprop.}); 129.6 (CH_{Ölsäure}); 129.9 (CH_{Ölsäure}); 173.1 (NCO); 173.1 (CO_{Ölsäure}); 173.3 (CO_{Ölsäure}).

### Beispiel 22

### Synthese von 2,3-Dioleoyloxy-N-(N-(N,N'N",N"'-Tetra-tert.-butyloxycarbonyl-6-carboxy-spermyl)-glycyl)-aminopropan 22

100 mg (0.15 mmol) **21** wurden zusammen mit 102 mg (0.16 mmol) (Boc)₄-Spermyl COOH und 39 mg (0.19 mmol) DCC in 1 ml CH₂Cl₂ gelöst und 18 h gerührt. Danach wurde von ausgefallenem DCH abfiltriert. Das Einengen und chromatographischer Aufreinigung (CH₂Cl₂/MeOH, 97:3) erhielt man 123.1 mg (63 %) **22** eines farblosen Öls.
^{**1**}**H-NMR (CDCl**_{**3**} **+ 0.1 ml CD**_{**3**}**COOD)**: δ = 0.88 (tr; 6 H; CH_{3,Ölsäure}); 1.28 (br s; 40 H; C-CH₂-C_{Ölsäure}); 1.46 (br s; CH_{3,Boc}; CH_{2,Spermyl}); 1.62 (m; 4 H; CH₂CH₂C=O); 2.03 (m; 8 H; CH₂-CH=CH); 2.32 (m; 4 H; CH₂C=O); 3.00-3.33 (m; 10 H; CH_{2,Spermyl}); 3.33-3.62 (m; 2 H; CH₂-N_{Aminoprop.}); 3.95 (m; 2 H; CH₂-N_{Gly}); 4.10 (m; 1 H; CH₂-O); 4.28 (m; 1 H; CH₂-O); 4.30 (br s; 1 H; CH_{Spermyl}); 5.12 (m; 1 H; CH_{Aminoprop.}); 5.35 (m; 4 H; CH=CH).
^{**13**}**C-NMR (CDCl**_{**3**} **+ 0.1 ml CD**_{**3**}**COOD):** δ = 14. (CH_{3,Ölsäure}); 22.8 (CH_{2,Ölsäure}); 25.0 (CH_{2,Ölsäure}); 27.3 (CH_{2,Ölsäure}); 27.3 (CH_{2,Ölsäure}); 28.4 (CH_{3,Boc}); 28.5 (CH_{3,Boc}); 29.3 (CH_{2,Ölsäure}); 29.3 (CH_{2,Ölsäure}); 29.4 (CH_{2,Ölsäure}); 29.7 (CH_{2,Ölsäure}); 29.9 (CH_{2,Ölsäure}); 32.0 (CH_{2,Ölsäure}); 34.2 (CH_{2,Ölsäure}); 39.7 (CH₂-N_{Gly}); 43.1 (CH₂-N_{Aminoprop.}); 63.0 (CH₂-O); 70.2 (CH-O); 79.6 (quart. C_{Boc}); 80.2 (quart. C_{Boc}); 80.9 (quart. C_{Boc}); 81.5 (quart. C_{Boc}); 129.8 (CH=CH); 130.1 (CH=CH); 155-158 (NCO₂); 173.6 (COO); 173.8 (COO).

### Beispiel 23

### Synthese von 2,3-Dioleoyloxy-N-(N-(6-carboxy-spermyl)-glycyl)-aminopropan 23

1.00 g (0.77 mmol) 22 wurden in 100 ml CH₂Cl₂/TFA (3:1) gelöst und 30 min gerührt. Anschließend wurde vom Lösungsmittel befreit. Man erhielt 1.03 g (98.80 %) 23 als wachsartiges Produkt.
^{**1**}**H-NMR (d**_{**6**}**-DMSO)**: δ = 0.85 (tr; 6 H; CH_{3,Ölsäure}); 1.23 (br s, 44 H, C-CH₂-C_{Ölsäure/Spermyl}); 1.50 (m; 4 H; CH₂CH₂C=O); 1.67 (m; 2 H; C-CH₂-C_{Spermyl}); 1.83 (m; 2 H; C-CH₂-C_{Spermyl}); 1.95 (m; 8 H; CH₂-CH=CH); 2.23 (m; 4 H; CH₂C=O); 2.82-3.30 (m; 10 H; N-CH_{2,Spermyl}); 3.26 (m; 1 H; CH₂-N_{Aminoprop.}); 3.36 (m; 1 H, CH₂-N_{Aminoprop.}); 3.74-3.88 (m; 2 H, CH₂-N_{Gly}); 3.93 (br s; 1 H; CH_{Spermyl}); 4.01 (m; 1 H, CH₂-O); 4.24 (m; 1 H; CH₂-O); 5.02 (m; 1 H; CH_{Aminoprop.}); 5.31 (m; 4 H; CH=CH); 8.10 (br s; 6 H; NH); 8.33 (tr; 1 H; NHC=O); 8.98 (br s; 2 H, NH); 9.04 (tr; 1 H; NHC=O); 9.15 (br s; 1 H; NH); 9.50 (br s; 1 H; NH).
^{**13**}**C-NMR (d**_{**6**}**-DMSO)**: δ = 13.8 (CH_{3,Ölsäure}); 20.9 (CH_{2,Ölsäure}); 22.1 (CH_{2,Ölsäure}); 23.8. 23.9 (CH_{2,Sper}); 24.4, 26.6 (CH_{2,Ölsäure}); 26.9 (CH_{2,Sper}); 28.5-29.1 (CH_{2,Ölsäure}); 31.3 (CH_{2,Ölsäure}); 33.4, 33.6 (CH_{2,Ölsäure}); 36.2 (CH_{2,Sper}); 38.7 (CH_{2,Sper}); 41.8 (CH₂-N_{Gly}); 43.0 CH₂-N_{Aminoprop.}); 43.9, 46.2 (CH_{2,Sper}); 58.8 (CH_{Sper}); 62.8 (CH₂-O); 129.5 (CH=CH); 129.6 (CH=CH); 167.4, 168,5 (NCO_{Sper/Gly}); 172.2 (COO); 172.4 (COO).

### Beispiel 24

### Transfektion von adhärenten HELA-Zellen mit CAT-Plasmid

### 1 Ausgangsmaterial

1. Medium für die Stammkultur HELA (ATTC-Nr. CCL 2): MEM (with EARL's Salts), 10 % FCS, 2 mM Pyruvat, 2 mM Glutamin, 1 x n.e. Aminosäuren.
   Für Transfektion gleiches Medium mit 5 % FCS und gleiches Medium ohne FCS ansetzen.
2. pSV2-CAT-Plasmid, in TE-Puffer, Konzentration: 1 mg/ml, 5 kb. (Gorman C.M. et al., Mol. Cell. Biol. 2, 1044-1051 (1982)).
3. Neue Transfektionsreagenzien:
   in MES (MES, 20 mM; NaCl, 150 mM; pH 6.2), Konzentration 1.0 mg/ml, beschallt (Branson-Sonifier) und sterilfiltriert
   oder
   in 99.8%igem EtOH, Konzentration 2.5 mg/ml (nicht beschallt und nicht steril filtriert)
4. HEPES: Hepes, 20 mM; NaCl, 150 mM, pH 7.4, steril
5. PBS, BM-Ident.-Nr. 210 331 (10 mM Puffer, 150 mM Salz), steril und nicht steril
6. Nacl, 150 mM
7. Lysispuffer: MOPS, 10 mM; NaCl, 10 mM, EGTA. 1 mM. Triton-X-100, 1 %. pH 6.5.

### 2. Transfektionsansatz

Einen Tag vor Transfektion werden die Zellen in 6 cm Petrischalen umgesetzt: Dazu Zellen abtrypsinisieren und auf 2 x 10⁵ Zellen/ml (Zellzahlbestimmung mit der Neubauer-Zählkammer) in 5 % FCS-haltigem Medium verdünnen. 5 ml pro Schale. Inkubation im Brutschrank (bei 37 °C, 5 % CO₂).

### Ansatz pro Schale für wäßrige Lösungen des Transfektionsreagenzes (TR):

1. 5 µg Plasmid (= 5 µl) mit HEPES ad 100 µl versetzen, vortexen.
2. 10 - 40 µg TR (= 10- 40 µl) mit HEPES ad 100 µ versetzen, vortexen.
3. Lösungen aus 1. und 2. vereinigen, vorsichtig schütteln. 10 bis 15 min bei RT stehen lassen.
4. Während der Inkubationszeit Mediumwechsel in den Testschalen: Altes Medium absaugen und durch 3 ml Medium (5% FCS-haltig oder falls ohne FCS, Zellen 2 x mit PBS waschen) ersetzen.
5. Plasmid-TR-Gemisch aus 3. (200 µl) direkt ins frische Medium (mit oder ohne FCS) geben und durch vorsichtiges Schwenken der Schale gleichmäßig verteilen.
6. 6 h im Brutschrank (bei 37 °C, 5 % CO₂) inkubieren.
7. Dann das Medium auf eine Endkonzentration von FCS von 5 - 10 % aufstocken. Mediumendvolumen 6 ml.
8. 19 h im Brutschrank inkubieren.
9. Dann Ansatz komplett absaugen und durch 5 ml Medium mit 5 - 10 % FCS ersetzen.

### Ansatz pro Schale für ethanolische Lösungen des TR:

### A Ohne FCS im Medium:

1. 5 µg Plasmid (= 5 µl) + 500 µl Medium ohne FCS. vortexen.
2. 10 - 40 µg TR (= 4- 16 µl) + 500 µl Medium ohne FCS. vortexen.
3. Zellen FCS-frei waschen: 5 ml Medium absaugen und x mit PBS waschen; absaugen.
4. Lösungen aus 1. und 2. vereinigen, vorsichtig schütteln.
5. Plasmid-TR-Gemisch aus 4. (ca. 1020 µl) auf die gewaschenen Zellen geben und durch vorsichtiges Schwenken der Schale gleichmäßig verteilen.
6. 6 h im Brutschrank (bei 37 °C, 5 % CO₂) inkubieren.
7. Dann das Medium auf eine Endkonzentration von FCS von 5 - 10 % aufstocken. Mediumend
8. 18 h im Brutschrank inkubieren.
9. Dann Ansatz komplett absaugen und durch 5 ml Medium mit 5 - 10 % FCS ersetzen.

### B Mit FCS im Medium:

1. 5 µg Plasmid (= 5 µl) + 150 µl NaCl, vortexen.
2. 10 - 40 µg TR (= 4 - 16 µl) + 150 µl NaCl, vortexen.
3. Lösungen aus 1. und 2. vereinigen, vorsichtig kurz schütteln. 15 min bei RT stehen lassen.
4. Während der Inkubationszeit Mediumwechsel in den Testschalen: Altes Medium absaugen und durch 1.5 ml Medium (10 % FCS-haltig) ersetzen.
5. Plasmid-TR-Gemisch aus 3. (ca. 320 µl) ins frische Medium geben und durch vorsichtiges Schwenken der Schale gleichmäßig verteilen.
6. 6 h im Brutschrank (bei 37 °C, 5 % CO₂) inkubieren.
7. Dann das Medium auf eine Endkonzentration von FCS von 5 - 10 % aufstocken. Mediumendvolumen 6 ml.
8. 18 h im Brutschrank inkubieren.
9. Dann Ansatz komplett absaugen und durch 5 ml Medium mit 5 - 10 % FCS ersetzen.

### 3. Zell-Lyse

1. Absaugen des Mediums und -3maliges Waschen der Zellen mit eiskaltem PBS.
2. Restflüssigkeit sorgfältig absaugen und 1 ml Lysispuffer pro Schale zugeben. Inkubation 30 min bei RT. Schalen ohne Schütteln stehen lassen.
3. Abnehmen des Zell-Lysates mit der Pipette und Überführen in 1.5 ml Eppendorftubes.
4. Abzentrifugieren der Lysate in der Tischzentrifuge, 3 min.
5. Überstand abnehmen und in ein frisches Gefäß überführen. Pellet verwerfen.

### 4. Proteinbestimmung der Lysate

Die Proteinbestimmung wird im Anschluß an die Lyse durchgeführt.

Analysiert wird mit der Proteinbestimung nach Bradford (M. Bradford, Anal. Biochem. 72, 248 (1976)).

Für die weitere Untersuchung werden die Lysate eines Versuchsansatzes mit Samplebuffer (aus CAT-ELISA-Kit, Boehringer Mannheim, Kat.-Nr. 1363727) auf die gleiche Proteinkonzentration eingestellt (ca. 250 µg/ml).

### 5. Ermittlung der Transfektionseffizienz

Untersuchung der Lysate im CAT-ELISA (Boehringer Mannheim, Kat.-Nr. 1363 727). Es werden je 200 µl der auf die gleiche Proteinkonzentration eingestellten Lysate im ELISA eingesetzt.

Vergleich der Extinktionen ergibt Aufschluß über die Transfektionseffizienz.

### Rekonstitution der Kit-Bestandteile und Verdünnen auf Arbeitskonzentration:

### 1. CAT-Standard, Flasche 1

Zu einer Flasche Lyophilisat 0,5 ml bidest. Wasser geben und durch Schütteln lösen. Die Konzentration (ng/ml) wird dem Etikettenaufdruck entnommen.

### Arbeitsverdünnung:

Gelöstes Lyophilisat (Konzentration siehe Flaschenetikett) mit Probenverdünnungspuffer (Flasche 7) auf 1.0, 0.5, 0.25. 0.125. 0.0625, 0.0312, 0 ng/ml dünnen.

### 2. PAK <CAT> Dig, Flasche Z

Zu einer Flasche Lyophilisat 0,5 ml bidest. Wasser geben und durch Schütteln lösen ( Konzentration = 0.2 mg/ml).

### Arbeitsverdünnung:

Das gelöste Lyophilisat (0.2 mg/ml) mit Probenverdünnungspuffer (Flasche 7) auf 2 µg/ml verdünnen.

### 3. PAK<Dig>POD, Flasche 3

Zu einer Flasche Lyophilisat 0.5 ml bidest. Wasser geben und durch Schütteln lösen (Konzentration = 20 U/ml).

### Arbeitsverdünnung:

Das gelöste Lyophilisat (20 U/ml) mit Probenverdünnungspuffer (Flasche 7) auf 150 mU/ml verdünnen.

### 4. Waschpuffer, Flasche 6

Gebrauchsfertiger Waschpuffer wird durch mischen von 1 Teil 10 x Waschpuffer (Fl. 6) mit 9 Teilen bidest. Wasser hergestellt. Der 1 x Waschpuffer wird für alle Waschschritte benötigt.

### 5. Probenverdünnungspuffer, Flasche 7

Im Wasserbad Flasche-Nr. 7 auf RT bringen. Ready to use.

### 6. POD-Substrat, Flasche 4

ABTS-1-Komponentensubstrat (Flasche Nr. 4), Ready to use, auf RT temperieren.

### Durchführung

Die nachfolgende Tabelle bezieht sich auf die o. g. Puffer und Arbeitsverdünnungen. Die MTP-Module werden nach jedem Waschschritt auf mehrfachen Cellulosepapierschichten ausgeklopft.

Für die Inkubationsschritte bei 37 °C wird die Platte mit einer MTP-Abdeckfolie beklebt.

| | **Arbeitsschritt** | **Volumen** | **Zeit** | **Temp.** | **Schütteln** |
|---|---|---|---|---|---|
| 1. | MTP-Modulstreifen entnehmen | | | | - |
| 2. | CAT-Enzym-Standard, Arbeitsverdünnungen oder Zell-Lysate | 200 µl/well | 60 min | 37 °C | - |
| 3. | Ausschütten, 3 x waschen mit Waschpuffer, ausklopfen | 300 µl/well | | 18-25 °C | - |
| 4. | PAK <CAT> DIG Arbeitsverdünnung | 200 µl/well | 60 min | 37 °C | - |
| 5. | Ausschütten, 3 x waschen mit Waschpuffer, ausklopfen | 300 µl/well | | 18-25 °C | - |
| 6. | PAK <DIG> POD Arbeitsverdünnung | 200 µl/well | 60 min | 37 °C | - |
| 7. | Ausschütten, 3 x waschen mit Waschpuffer, ausklopfen | 300 µl/well | | 18-25 °C | - |
| 8. | Substratlösung | 200 µl/well | 10 min | 18-25 °C | + |
| 9. | Messung bei 405 nm nach ca. 10 min Substratinkubation | | | | |

Die Ergebnisse sind in Abbildungen 1 und 2 dargestellt.

## Patentansprüche

1. Verbindungen der allgemeinen Formel (I) 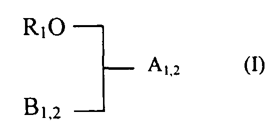 wobei
R₁ eine gesättigte oder ungesättigte C(O)-Cl₋₂₃ oder gesättigte oder ungesättigte C₁₋₂₄-Kette darstellt.
A₁ eine O-R₂-Gruppe, wobei R₂ die für R₁ angegebene Bedeutung hat und gleich oder von R₁ verschieden sein kann.
A₂ einen NR₃X- oder einen N⁺R₃R₄R₅Y⁻-Rest, wobei
R₃, R₄, die untereinander gleich oder verschieden sein können mit der Maßgabe, daß R₃ und R₄ nicht gleichzeitig Wasserstoff sein können, Wasserstoff, eine Alkylgruppe mit 1 bis 4 C-Atomen, eine (CH₂)ₙ -OH- oder eine (CH₂)ₙ-NH₂-Gruppe mit n = 2-6 bedeuten,
R₅, welcher gleich oder verschieden von R₃ oder R₄ sein kann, Wasserstoff, eine Alkylgruppe mit 1 bis 4 C-Atomen, eine (CH₂)ₙ -OH-, eine (CH₂)ₙ-Halogenid-, oder eine ((CH₂)ₘNH)ₒ-(CH₂)ₙ -NH₂-Gruppe bedeuten, wobei m eine ganze Zahl von 2 bis 6 ist und gleich oder verschieden von n oder o sein kann, wobei n eine Zahl von 2 bis 6 und o eine ganze Zahl von 0 bis 4 sein kann,
X zusätzlich zu der Bedeutung für R₅, folgende Bedeutung haben kann: eine amidisch gebundene Aminosäure, ein amidisch gebundenes Peptid bzw. Polypeptid, eine -C(O)-CHR₆N(R₇)₂-, eine -C(O)-CHR₆N⁺(R₇)₃-, eine C(O)-CHR₆N⁺(R₇)₂R₈- oder eine -C(O)-CHR₆N R₇R₈-Gruppe mit der Maßgabe, daß wenn R₃ Wasserstoff ist, X eine amidisch gebundene Aminosäure, ein Aminosäurederivat ein Peptid bzw. Polypeptid bedeutet, wobei
R₆ ein -(CH₂)ₘ-NR₇R₈-, ein -(CH₂)ₘ -N⁺(R₇)₃- oder ein (CH₂)ₘ-N⁺(R₇)₂R₈-Rest und m eine Zahl von 1 bis 5 sein kann,
R₇ Wasserstoff oder eine Alkylgruppe mit 1 bis 4 C-Atomen,
R₈ eine -(CH₂)ₙ -N(R₇)₂- oder -(CH₂)ₙ -N⁺(R₇)₃ -Gruppe, wobei n eine Zahl von 2 bis 4 ist und R₇ die oben angegebene Bedeutung haben kann und
Y ein pharmazeutisch akzeptables Anion ist.
B₁ ein NH[C(O)-(CH₂)p -NH]q - Z -Rest, wobei p eine Zahl von 1 bis 6 und q eine Zahl von 0 bis 2 ist,
Z eine amidisch gebundene Aminosäure, ein amidisch gebundenes Peptid bzw. Polypeptid eine -C(O)-CHR₆N (R₇)₂-, eine -C(O)-CHR₆N⁺(R₇)₃-, eine C(O)-CHR₆N⁺(R₇)₂ R₈- oder eine -C(O)-CHR₆NR₇R₈-Gruppe darstellen und R₆ bis R₈ und m die oben angegebenen Bedeutungen haben,
und
B₂ eine O-R₂-Gruppe, wobei R₂ die für R₁ angegebene Bedeutung hat und gleich oder von R₁ verschieden sein kann,
mit der Maßgabe, daß die Bedeutung von A₁ nur mit B₁, die von A₂ nur mit B₂ gilt.

2. Verbindungen nach Anspruch 1. dadurch gekennzeichnet, daß der Rest R₁ eine gesättigte oder ungesättigte C₁₀₋₂₀- oder C(O)₁₀₋₂₀-Gruppe darstellt.

3. Verbindungen nach Anspruch 1 oder 2, dadurch gekennzeichnet, daß die Bedeutung von A₂NR₃X mit R₃ Wasserstoff und X eine amidisch gebundene Aminosäure, ein Aminosäurederivat, ein Peptid bzw. Polypeptid ist.

4. Verbindungen der allgemeinen Formel (I) 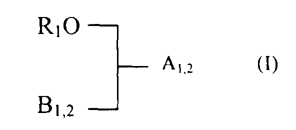 wobei
R₁ eine gesättigte oder ungesättigte C(O)-Cl₋₂₃ oder gesättigte oder ungesättigte C₁₋₂₄-Kette darstellt.
A₁ eine O-R₂-Gruppe. wobei R₂ die für R₁ angegebene Bedeutung hat und gleich oder von R₁ verschieden sein kann.
A₂ einen NR3X- oder einen N⁺R₃R₄R₅Y⁻-Rest, wobei
R₃, R₄, die untereinander gleich oder verschieden sein können, Wasserstoff, eine Alkylgruppe mit 1 bis 4 C-Atomen, eine (CH₂)ₙ -OH- oder eine (CH₂)ₙ-NH₂-Gruppe mit n = 2-6 bedeuten,
R₅, welcher gleich oder verschieden von R₃ oder R₄ sein kann, Wasserstoff, eine Alkylgruppe mit 1 bis 4 C-Atomen. eine (CH₂)ₙ -OH-, eine (CH₂)ₙ-Halogenid-, oder eine ((CH₂)ₘNH)ₒ-(CH₂)ₙ -NH₂-Gruppe bedeuten, wobei m eine ganze Zahl von 2 bis 6 ist und gleich oder verschieden von n oder o sein kann, wobei n eine Zahl von 2 bis 6 und o eine ganze Zahl von 0 bis 4 sein kann,
X zusätzlich zu der Bedeutung für R₅, folgende Bedeutung haben kann: eine amidisch gebundene Aminosäure, ein amidisch gebundenes Peptid bzw. Polypeptid, eine -C(O)-CHR₆N(R₇)₂-, eine -C(O)-CHR₆N⁺(R₇)₃-, eine C(O)-CHR₆N⁺(R₇)₂R₈- oder eine -C(O)-CHR₆N R₇R₈-Gruppe wobei
R₆ ein -(CH₂)ₘ-NR₇R₈-, ein -(CH₂)ₘ -N⁺(R₇)₃- oder ein (CH₂)ₘ-N⁺(R₇)₂R₈-Rest und m eine Zahl von 1 bis 5 sein kann,
R₇ Wasserstoff oder eine Alkylgruppe mit 1 bis 4 C-Atomen,
R₈ eine -(CH₂)ₙ -N(R₇)₂- oder -(CH₂)ₙ -N⁺(R₇)₃ -Gruppe, wobei n eine Zahl von 2 bis 4 ist und R₇ die oben angegebene Bedeutung haben kann und
Y ein pharmazeutisch akzeptables Anion ist.
B₁ ein NH(C(O)-(CH₂)ₚ-NH)_{q}-Z-Rest, wobei p die Zahl 1 oder 5, q 0 oder 1 und Z eine -C(O)-CHR₆N(R7)₂-, eine -C(O)-CHR₆N⁺(R7)₃-, eine -C(O)-CHR₆N⁺(R7)₂R₈-Gruppe ist und R₆ bis R₈ die zuvor angegebenen Bedeutungen haben
B₂ eine O-R₂-Gruppe, wobei R₂ die für R₁ angegebene Bedeutung hat und gleich oder von R₁ verschieden sein kann,
mit der Maßgabe, daß die Bedeutung von A₁ nur mit B₁, die von A₂ nur mit B₂ gilt.

5. Verbindungen nach Anspruch 1, 2, 3 oder 4, dadurch gekennzeichnet, daß Y ein Halogenid, Monomethylsulfat, Acetat, Trifluoroacetat oder Phosphat bedeutet.

6. Verbindung nach einem der Ansprüche 1 bis 5, dadurch gekennzeichnet, daß es sich um 2-(6-Carboxyspermyl)-1,3-dioleoyloxy-propylamid, 1-(6-Carboxyspermyl)-2,3-dioleoyloxy-propylamid, 2,3-Dioleoyloxy-N-(N-(6-carboxyspermyl)-glycyl)-aminopropan, 2-(6-Carboxyspermyl)-1,3-dimyristoyloxy-propylamid, 2-(1,1,1,5,5,10, 10,14,14,14-Deca-methyl-6-carboxylspermyl)-1,3-dioleoyloxy-propylamid, 2-(N,N,N,N',N',N'-Hexamethylomithyl)-1,3-dioleoyloxy-propylamid und/oder 2-( N,N,N,N',N',N'-Hexamethyllysyl)-1,3-dioleoyloxy-propylamid handelt.

7. Reagenz bestehend aus mindestens einer Verbindung der allgemeinen Formel (I), dadurch gekennzeichnet, daß die Lösung ein wassermischbares Lösungsmittel enthält und Formel (I) wie folgt definiert ist: 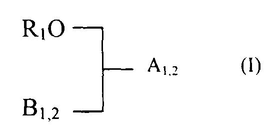 wobei
R₁ eine gesättigte oder ungesättigte C(O)-Cl₋₂₃ oder gesättigte oder ungesättigte C₁₋₂₄-Kette darstellt.
A₁ eine O-R₂-Gruppe, wobei R₂ die für R₁ angegebene Bedeutung hat und gleich oder von R₁ verschieden sein kann.
A₂ einen NR₃X- oder einen N⁺R₃R₄R₅Y⁻-Rest, wobei
R₃, R₄, die untereinander gleich oder verschieden sein können, Wasserstoff, eine Alkylgruppe mit 1 bis 4 C-Atomen, eine (CH₂)ₙ -OH- oder eine (CH₂)ₙ-NH₂-Gruppe mit n = 2-6 bedeuten.
R₅, welcher gleich oder verschieden von R₃ oder R₄ sein kann, Wasserstoff, eine Alkylgruppe mit 1 bis 4 C-Atomen, eine (CH₂)ₙ -OH-, eine (CH₂)ₙ-Halogenid-, oder eine ((CH₂)ₘNH)ₒ-(CH₂)ₙ -NH₂-Gruppe bedeuten, wobei m eine ganze Zahl von 2 bis 6 ist und gleich oder verschieden von n oder o sein kann, wobei n eine Zahl von 2 bis 6 und o eine ganze Zahl von 0 bis 4 sein kann,
X zusätzlich zu der Bedeutung für R₅, folgende Bedeutung haben kann: eine amidisch gebundene Aminosäure, ein amidisch gebundenes Peptid bzw. Polypeptid, eine -C(O)-CHR₆N(R₇)₂-, eine -C(O)-CHR₆N⁺(R₇)₃-, eine C(O)-CHR₆N⁺(R₇)₂R₈- oder eine -C(O)-CHR₆N R₇R₈-Gruppe wobei
R₆ ein -(CH₂)ₘ-NR₇R₈-, ein -(CH₂)ₘ -N⁺(R₇)₃- oder ein (CH₂)ₘ-N⁺(R₇)₂R₈-Rest und m eine Zahl von 1 bis 5 sein kann,
R₇ Wasserstoff oder eine Alkylgruppe mit 1 bis 4 C-Atomen,
R₈ eine -(CH₂)ₙ -N(R₇)₂- oder -(CH₂)ₙ -N⁺(R₇)₃ -Gruppe, wobei n eine Zahl von 2 bis 4 ist und R₇ die oben angegebene Bedeutung haben kann und
Y ein pharmazeutisch akzeptables Anion ist.
B₁ ein NH[C(O)-(CH₂)p -NH]q - Z -Rest, wobei p eine Zahl von 1 bis 6 und q eine Zahl von 0 bis 2 ist,
Z eine amidisch gebundene Aminosäure, ein amidisch gebundenes Peptid bzw. Polypeptid eine -C(O)-CHR₆N (R₇)₂-, eine -C(O)-CHR₆N⁺(R₇)₃-, eine C(O)-CHR₆N⁺(R₇)₂ R₈- oder eine -C(O)-CHR₆NR₇R₈-Gruppe darstellen und R₆ bis R₈ und m die oben angegebenen Bedeutungen haben,
und
B₂ eine O-R₂-Gruppe, wobei R₂ die für R₁ angegebene Bedeutung hat und gleich oder von R₁ verschieden sein kann,
mit der Maßgabe, daß die Bedeutung von A₁ nur mit B₁, die von A₂ nur mit B₂ gilt.

8. Reagenz nach Anspruch 7 dadurch gekennzeichnet, daß die Lösung ein Gemisch bestehend aus mindestens einer Verbindung gemäß der allgemeinen Formel (1) und einer anderen lipidischen Verbindung in wassermischbaren Lösungsmitteln enthält.

9. Reagenz nach Anspruch 7 oder 8, dadurch gekennzeichnet, daß es sich bei dem wasser-mischbaren Lösungsmittel um einen niedrigen Alkohol handelt.

10. Reagenz dadurch gekennzeichnet, daß eine oder mehrere Verbindungen der Ansprüche 1 bis 6 gegebenenfalls in Gegenwart anderer lipidischer Verbindungen in Form von Liposomen oder anderen Aggregaten vorliegen.

11. Verfahren zur Einschleusung von (Bio-)Molekülen in Zellen, dadurch gekennzeichnet, daß ein Reagenz der Ansprüche 7 bis 10 verwendet wird und dieses zuerst mit einem anionischen Biomolekül und anschließend zur Transfektion mit Zellen in Kontakt gebracht wird.

12. Verfahren nach Anspruch 11, dadurch gekennzeichnet, daß es sich bei den einzuschleusenden Molekülen um DNA oder RNA handelt.

13. Verfahren nach Anspruch 11, dadurch gekennzeichnet, daß das anionische Biomolekül DNA oder ein entsprechendes Fragment ist.

14. Verfahren nach Anspruch 11 bis 13, dadurch gekennzeichnet, daß 2-(6-carboxyspermyl)-1,3-dioleoyloxy-propylamid, 1-(6-Carboxyspermyl)-2,3-dioleoyloxy-propylamid, 2,3-dioleoyloxy-N-(N-(6-Carboxyspermyl)-glycyl)-aminopropan, 2-(6-carboxyypermyl)-1,3-dimyristoyloxy-propylamid, 2-(1,1,1,5,5,10,10,14,14,14-Decamethyl-6-carboxylspermyl)-1,3-dioleoyloxy-propylamid, 2-(N,N,N,N',N',N'-Hexamethylornithyl)-1,3-dioleoyloxy-propylamid und/oder 2-( N,N,N,N',N',N'-Hexamethyllysyl)-1,3-dioleoyloxy-propylamid alleine oder gegebenenfalls in Gegenwart einer oder mehrerer anderer lipidischer Verbindungen verwendet wird.

## Claims

1. Compounds of the general formula (I) 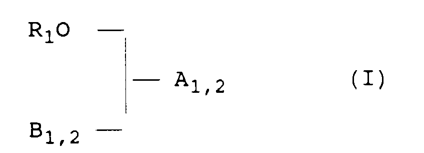 in which
R₁ represents a saturated or unsaturated C(O)-C₁₋₂₃ or saturated or unsaturated C₁₋₂₄ chain,
A₁ represents an O-R₂ group in which R₂ has the meaning stated for R₁ and can be the same as or different to R₁,
A₂ represents an NR₃X or an N⁺R₃R₄R₅Y⁻ residue in which
R₃, R₄ which are the same as or different to one another, represent hydrogen, an alkyl group with 1 to 4 C atoms, a (CH₂)ₙ-OH or a (CH₂)ₙ-NH₂ group where n = 2-6, provided that R₃ and R₄ cannot both be hydrogen,
R₅ which can be the same as or different to R₃ or R₄, denotes hydrogen, an alkyl group with 1 to 4 C atoms, a (CH₂)ₙ-OH, a (CH₂)ₙ-halogenide or a ((CH₂)ₘNH)ₒ-(CH₂)ₙ-NH₂ group in which m is an integer from 2 to 6 and can be the same or different to n or o, wherein n can be a number from 2 to 6 and o an integer from 0 to 4,
X can in addition to the meaning for R₅ have the following meaning: an amidically bound amino acid, an amidically bound peptide or polypeptide, a C(O)-CHR₆N(R₇)₂, a C(O)-CHR₆N⁺(R₇)₃, a C(O)-CHR₆N⁺(R₇)₂R₈ or a C(O)-CHR₆NR₇R₈ group provided that if R₃ is hydrogen, X denotes an amidically bound amino acid, an amino acid derivative, a peptide or polypeptide, in which
R₆ can be a (CH₂)ₘ-NR₇R₈, a (CH₂)ₘ-N⁺(R₇)₃ or a (CH₂)ₘ-N⁺(R₇)₂R₈ residue and m can be a number from 1 to 5,
R₇ represents hydrogen or an alkyl group with 1 to 4 C atoms,
R₈ represents a (CH₂)ₙ-N(R₇)₂ or (CH₂)ₙ-N⁺(R₇)₃ group in which n is a number of 2 to 4 and R₇ can have the meaning stated above and
Y is a pharmaceutically acceptable anion,
B₁ represents an NH[C(O)-(CH₂)ₚ-NH]_{q}-Z residue in which p is a number from 1 to 6 and q is a number from 0 to 2,
Z represents an amidically bound amino acid, an amidically bound peptide or polypeptide, a C(O)-CHR₆N(R₇)₂, a C(O)-CHR₆N⁺(R₇)₃, a C(O)-CHR₆N⁺(R₇)₂R₈ or a C(O)-CHR₆NR₇R₈ group and R₆ to R₈ and m have the aforementioned meanings
and
B₂ represents an O-R₂ group in which R₂ has the meaning stated for R₁ and can be the same as or different to R₁ provided that the meaning of A₁ is only valid with B₁ and that of A₂ is only valid with B₂.

2. Compounds as claimed in claim 1, wherein the residue R₁ represents a saturated or unsaturated C₁₀₋₂₀ or C(O)₁₀₋₂₀ group.

3. Compounds as claimed in claim 1 or 2, wherein the meaning of A₂ is NR₃X where R₃ denotes hydrogen and X denotes an amidically bound amino acid, an amino acid derivative, a peptide or polypeptide.

4. Compounds of the general formula (I) 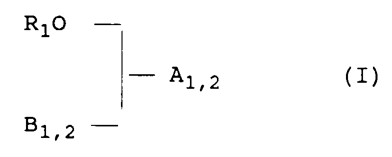 in which
R₁ represents a saturated or unsaturated C(O)-C₁₋₂₃ or saturated or unsaturated C₁₋₂₄ chain,
A₁ represents an O-R₂ group in which R₂ has the meaning stated for R₁ and can be the same as or different to R₁,
A₂ represents an NR₃X or an N⁺R₃R₄R₅Y⁻ residue in which
R₃, R₄ which can be the same as or different to one another, represent hydrogen, an alkyl group with 1 to 4 C atoms, a (CH₂)ₙ-OH or a (CH₂)ₙ-NH₂ group where n = 2-6,
R₅ which can be the same as or different to R₃ or R₄, denotes hydrogen, an alkyl group with 1 to 4 C atoms, a (CH₂)ₙ-OH, a (CH₂)ₙ-halogenide or a ((CH₂)ₘNH)ₒ-(CH₂)ₙ-NH₂ group in which m is an integer from 2 to 6 and can be the same or different to n or o, wherein n can be a number from 2 to 6 and o an integer from 0 to 4,
X can in addition to the meaning for R₅ have the following meaning: an amidically bound amino acid, an amidically bound peptide or polypeptide, a C(O)-CHR₆N(R₇)₂, a C(O)-CHR₆N⁺(R₇)₃, a C(O)-CHR₆N⁺(R₇)₂R₈ or a C(O)-CHR₆NR₇R₈ group, in which
R₆ can be a (CH₂)ₘ-NR₇R₈, a (CH₂)ₘ-N⁺(R₇)₃ or a (CH₂)ₘ-N⁺(R₇)₂R₈ residue and m can be a number from 1 to 5,
R₇ represents hydrogen or an alkyl group with 1 to 4 C atoms,
R₈ represents a (CH₂)ₙ-N(R₇)₂ or (CH₂)ₙ-N⁺(R₇)₃ group in which n is a number of 2 to 4 and R₇ can have the meaning stated above and
Y is a pharmaceutically acceptable anion,
B₁ denotes a NH(C(O)-(CH₂)ₚ-NH)_{q}-Z residue in which p denotes the number 1 or 5, q is 0 or 1 and Z is a -C(O)-CHR₆N(R₇)₂, a -C(O)-CHR₆N⁺(R₇)₃, a -C(O)-CHR₆N⁺(R₇)₂R₈ group and R₆ to R₈ have the meanings stated above,
B₂ represents an O-R₂ group in which R₂ has the meaning stated for R₁ and can be the same as or different to R₁
provided that the meaning of A₁ is only valid with B₁ and that of A₂ is only valid with B₂.

5. Compounds as claimed in claim 1, 2, 3 or 4, wherein Y denotes a halogenide, monomethyl sulfate, acetate, trifluoroacetate or phosphate.

6. Compound as claimed in one of the claims 1 to 5, wherein it is a 2-(6-carboxyspermyl)-1,3-dioleoyloxy-propylamide, 1-(6-carboxyspermyl)-2,3-dioleoyloxy-propylamide, 2,3-dioleoyloxy-N-(N-(6-carboxyspermyl)-glycyl)-aminopropane, 2-(6-carboxyspermyl)-1,3-dimyristoyloxy-propylamide, 2-(1,1,1,5,5,10,10,14, 14,14-deca-methyl-6-carboxyspermyl)-1,3-dioleoyloxy-propylamide, 2-(N,N,N,N',N',N'-hexamethylornithyl)-1,3-dioleoyloxy-propylamide and/or 2-(N,N,N,N',N',N'-hexamethyllysyl)-1,3-dioleoyloxy-propylamide.

7. Reagent composed of at least one compound of the general formula (I), wherein the solution contains a water-miscible solvent and formula (I) is defined as follows: 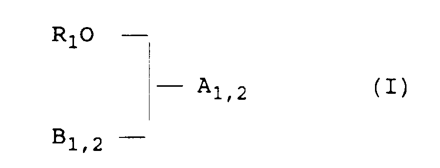 in which
R₁ represents a saturated or unsaturated C(O)-C₁₋₂₃ or saturated or unsaturated C₁₋₂₄ chain,
A₁ represents an O-R₂ group in which R₂ has the meaning stated for R₁ and can be the same as or different to R₁,
A₂ represents an NR₃X or an N⁺R₃R₄R₅Y⁻ residue in which
R₃, R₄ which can be the same as or different to one another, represent hydrogen, an alkyl group with 1 to 4 C atoms, a (CH₂)ₙ-OH or a (CH₂)ₙ-NH₂ group where n = 2-6,
R₅ which can be the same as or different to R₃ or R₄, denotes hydrogen, an alkyl group with 1 to 4 C atoms, a (CH₂)ₙ-OH, a (CH₂)ₙ-halogenide or a ((CH₂)ₘNH)ₒ-(CH₂)ₙ-NH₂ group in which m is an integer from 2 to 6 and can be the same or different to n or o, wherein n can be a number from 2 to 6 and o an integer from 0 to 4,
X can in addition to the meaning for R₅ have the following meaning: an amidically bound amino acid, an amidically bound peptide or polypeptide, a C(O)-CHR₆N(R₇)₂, a C(O)-CHR₆N⁺(R₇)₃, a C(O)-CHR₆N⁺(R₇)₂R₈ or a C(O)-CHR₆NR₇R₈ group, in which
R₆ can be a (CH₂)ₘ-NR₇R₈, a (CH₂)ₘ-N⁺(R₇)₃ or a (CH₂)ₘ-N⁺(R₇)₂R₈ residue and m can be a number from 1 to 5,
R₇ represents hydrogen or an alkyl group with 1 to 4 C atoms,
R₈ represents a (CH₂)ₙ-N(R₇)₂ or (CH₂)ₙ-N⁺(R₇)₃ group in which n is a number of 2 to 4 and R₇ can have the meaning stated above and
Y is a pharmaceutically acceptable anion,
B₁ represents an NH[C(O)-(CH₂)ₚ-NH]_{q}-Z residue in which p is a number from 1 to 6 and q is a number from 0 to 2,
Z represents an amidically bound amino acid, an amidically bound peptide or polypeptide, a C(O)-CHR₆N(R₇)₂, a C(O)-CHR₆N⁺(R₇)₃, a C(O)-CHR₆N⁺(R₇)₂R₈ or a C(O)-CHR₆NR₇R₈ group and R₆ to R₈ and m have the aforementioned meanings
and
B₂ represents an O-R₂ group in which R₂ has the meaning stated for R₁ and can be the same as or different to R₁
provided that the meaning of A₁ is only valid with B₁ and that of A₂ is only valid with B₂.

8. Reagent as claimed in claim 7, wherein the solution contains a mixture composed of at least one compound of the general formula (I) and another lipidic compound in water-miscible solvents.

9. Reagent as claimed in claim 7 or 8, wherein the water-miscible solvent is a lower alcohol.

10. Reagent wherein one or several compounds as claimed in claims 1 to 6 are present in the form of liposomes or other aggregates optionally in the presence of other lipidic compounds

11. Method for delivering (bio)molecules into cells, wherein a reagent of claims 7 to 10 is used and this is firstly contacted with an anionic biomolecule and subsequently with cells for the transfection.

12. Method as claimed in claim 11, wherein the molecules to be delivered are DNA or RNA.

13. Method as claimed in claim 11, wherein the anionic biomolecule is DNA or a corresponding fragment.

14. Method as claimed in claim 11 to 13, wherein 2-(6-carboxyspermyl)-1,3-dioleoyloxy-propylamide, 1-(6-carboxyspermyl)-2,3-dioleoyloxy-propylamide, 2,3-dioleoyloxy-N-(N-(6-carboxyspermyl)-glycyl)-amino-propane, 2-(6-carboxyspermyl)-1,3-dimyristoyloxy-propylamide, 2-(1,1,1,5,5,10,10,14,14,14-decamethyl-6-carboxyspermyl)-1,3-dioleoyloxy-propylamide, 2-(N,N,N,N',N',N'-hexamethylornithyl)-1,3-dioleoyloxy-propylamide and/or 2-(N,N,N,N',N',N'-hexamethyllysyl)-1,3-dioleoyloxy-propylamide are used alone or optionally in the presence of one or several other lipidic compounds.

## Revendications

1. Composés répondant à la Formule générale (I) 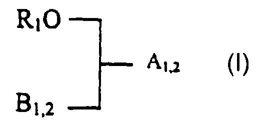 où
R₁ représente une chaîne C(O)-C₁₋₂₃ saturée ou insaturée ou une chaîne en C₁₋₂₄ saturée ou insaturée,
A₁ représente un groupe O-R₂, où R₂ présente la signification indiquée pour R₂, et peut être identique à ou différent de R₁,
A₂ représente un résidu NR₃X ou un résidu N⁺R₃R₄R₅Y⁻, où
R₃, R₄, qui peuvent être identiques ou différents entre eux sous condition que R₃ et R₄ ne peuvent pas représenter simultanément un atome d'hydrogène, signifient un atome d'hydrogène, un groupe alkyle avec 1 à 4 atomes de carbone, un groupe (CH₂)ₘ-OH ou un groupe (CH₂)ₘ-NH₂, avec n = 2- 6,
R₅, qui peut être identique à ou différent de R₃ ou de R₄, signifie un atome d'hydrogène, un groupe alkyle avec 1 à 4 atomes de carbone, un groupe (CH₂)ₙ-OH, un groupe (CH₂)ₙ-halogénure ou un groupe ((CH₂)ₘ-NH)ₒ-(CH₂)ₙ-NH₂, où m représente un nombre entier de 2 à 6 et peut être identique à ou différent de n ou de o, n pouvant être un nombre de 2 à 6 et o un nombre entier de 0 à 4,
X peut avoir comme signification outre celle donnée pour R₅ : un acide aminé lié par une liaison amidique, un peptide, respectivement polypeptide lié par une liaison amidique, un groupe -C(O)-CHR₆N(R₇)₂, un groupe -C(O)-CHR₆N⁺(R₇)₃, un groupe -C(O)-CHR₆N⁺(R₇)₂R₈ ou un groupe -C(O)-CHR₆NR₇R₈ sous condition que lorsque R₃ représente un atome d'hydrogène, X représente un acide aminé, dérivé d'acide aminé, peptide, respectivement polypeptide lié par une liaison amidique, où
R₆ peut représenter un résidu -(CH₂)ₘ-NR₇R₈, un résidu -(CH₂)ₘ-N⁺(R₇)₃ ou un résidu -(CH₂)ₘ-N⁺(R₇)₂R₈ et m peut représenter un nombre de 1 à 5,
R₇ représente un atome d'hydrogène ou un groupe alkyle avec 1 à 4 atomes de carbone,
R₈ peut représenter un groupe -(CH₂)ₙ-N(R₇)₂ ou un groupe -(CH₂)ₙ-N⁺(R₇)₃ où n est un nombre de 2 à 4 et R₇ peut présenter la signification indiquée ci-dessus et
Y est un anion pharmaceutiquement acceptable,
B₁ représente un résidu NH[C(O)-(CH₂)ₚ-NH]_{q}-Z où p représente un nombre de 1 à 6 et q un nombre de 0 à 2,
Z représente un acide aminé, peptide, respectivement polypeptide lié par une liaison amidique, un groupe -C(O)-CHR₆N(R₇)₂, un groupe -C(O)-CHR₆N⁺(R₇)₃, un groupe -C(O)-CHR₆N⁺(R₇)₂R₈ ou un groupe -C(O)-CHR₆NR₇R₈ et R₆ à R₈ et m présentent les significations indiquées ci-dessus,
et
B₂ représente un groupe O-R₂, où R₂ présente la signification indiquée pour R₁ et peut être identique à ou différent de R₁,
sous condition que la signification de A₁ ne vaut qu'avec B₁ et celle de A₂ qu'avec B₂.

2. Composés selon la revendication 1, caractérisés en ce que le résidu R₁ représente un groupe en C₁₀₋₂₀ ou un groupe C(O)₁₀₋₂₀ saturé ou insaturé.

3. Composés selon la revendication 1 ou 2, caractérisés en ce que la signification de A₂NR₃X est telle que R₃ représente un atome d'hydrogène et X un acide aminé, dérivé d'acide aminé, peptide, respectivement polypeptide lié par une liaison amidique.

4. Composés répondant à la Formule générale (I) 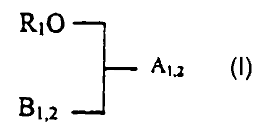 où
R₁ représente une chaîne C(O)-C₁₋₂₃ saturée ou insaturée ou une chaîne en C₁₋₂₄ saturée ou insaturée,
A₁ représente un groupe O-R₂, où R₂ présente la signification indiquée pour R₁, et peut être identique à ou différent de R₁,
A₂ représente un résidu NR₃X ou un résidu N⁺R₃R₄R₅Y⁻, où
R₃, R₄ qui peuvent être identiques ou différents entre eux, signifient un atome d'hydrogène, un groupe alkyle avec 1 à 4 atomes de carbone, un groupe (CH₂)ₘ-OH ou un groupe (CH₂)ₘ-NH₂, avec n = 2 - 6,
R₅, qui peut être identique à ou différent de R₃ ou de R₄, signifie un atome d'hydrogène, un groupe alkyle avec 1 à 4 atomes de carbone, un groupe (CH₂)ₙ-OH, un groupe (CH₂)ₙ-halogénure ou un groupe ((CH₂)ₘ-NH)ₒ-(CH₂)ₙ-NH₂, où m représente un nombre entier de 2 à 6 et peut être identique à ou différent de n ou de o, n pouvant être un nombre de 2 à 6 et o un nombre entier de 0 à 4,
X peut avoir comme signification outre celle donnée pour R₅: un acide aminé lié par une liaison amidique, un peptide, respectivement polypeptide lié par une liaison amidique, un groupe -C(O)-CHR₆N(R₇)₂, un groupe -C(O)-CHR₆N⁺(R₇)₃, un groupe -C(O)-CHR₆N⁺(R₇)₂R₈ ou un groupe -C(O)-CHR₆NR₇R₈, où
R₆ peut représenter un résidu -(CH₂)ₘ-NR₇R₈, un résidu -(CH₂)ₘ-N⁺(R₇)₃ ou un résidu -(CH₂)ₘ-N⁺(R₇)₂R₈ et m peut représenter un nombre de 1 à 5,
R₇ représente un atome d'hydrogène ou un groupe alkyle avec 1 à 4 atomes de carbone,
R₈ peut représenter un groupe -(CH₂)ₙ-N(R₇)₂ ou un groupe -(CH₂)ₙ-N⁺(R₇)₃ où n est un nombre de 2 à 4 et R₇ peut présenter la signification indiquée ci-dessus et
Y est un anion pharmaceutiquement acceptable,
B₁ représente un résidu NH(C(O)-(CH₂)ₚ-NH)_{q}-Z où p représente le nombre 1 ou 5, q 0 ou 1 et Z représente un groupe -C(O)-CHR₆N(R₇)₂, un groupe -C(O)-CHR₆N⁺(R₇)₃, un groupe -C(O)-CHR₆N⁺(R₇)₂R₈ et R₆ à R₈ présentent les significations indiquées ci-dessus,
B₂ représente un groupe O-R₂, où R₂ présente la signification indiquée pour R₁ et peut être identique à ou différent de R₁,
sous condition que la signification de A₁ ne vaut qu'avec B₁ et celle de A₂ qu'avec B₂.

5. Composés selon la revendication 1, 2, 3 ou 4, caractérisés en ce que Y signifie un halogénure, du monométhylsulfate, de l'acétate, du trifluoroacétate ou du phosphate.

6. Composé selon l'une des revendication 1 à 5, caractérisé en ce qu'il s'agit du 2-(6-carboxyspermyl)-1,3-dioléoyloxypropylamide, du 1-(6-carboxyspermyl)-2,3-dioléoyloxypropylamide, du 2,3-dioléoyloxy-N-(N-(6-carboxyspermyl)glycyl)aminopropane, du 2-(6-carboxyspermyl)-1,3-dimyristoyl-oxypropylamide, du 2-(1,1,1,5,5,10,10,14,14,14-décaméthyl-6-carboxyspermyl)-1,3-dioléoyloxypropylamide, du 2-(N,N,N,N',N',N'-hexaméthylornithyl)-1,3-dioléoyloxypropylamide et/ou du 2-(N,N,N,N',N',N'-hexaméthyllysyl)-1,3-dioléoyloxypropylamide.

7. Réactif constitué d'au moins un composé répondant à la Formule générale (I), caractérisé en ce que la solution contient un solvant miscible à l'eau et en ce que la Formule (I) est définie de la manière suivante : 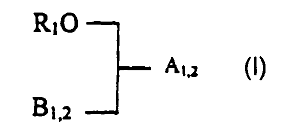 où
R₁ représente une chaîne C(O)-C₁₋₂₃ saturée ou insaturée ou une chaîne en C₁₋₂₄ saturée ou insaturée,
A₁ représente un groupe O-R₂, où R₂ présente la signification indiquée pour R₁, et peut être identique à ou différent de R₁,
A₂ représente un résidu NR₃X ou un résidu N⁺R₃R₄R₅Y⁻, où
R₃, R₄ qui peuvent être identiques ou différents entre eux, signifient un atome d'hydrogène, un groupe alkyle avec 1 à 4 atomes de carbone, un groupe (CH₂)ₘ-OH ou un groupe (CH₂)ₘ-NH₂, avec n = 2 - 6,
R₅, qui peut être identique à ou différent de R₃ ou de R₄, signifie un atome d'hydrogène, un groupe alkyle avec 1 à 4 atomes de carbone, un groupe (CH₂)ₙ-OH, un groupe (CH₂)ₙ-halogénure ou un groupe ((CH₂)ₘ-NH)ₒ-(CH₂)ₙ-NH₂, où m représente un nombre entier de 2 à 6 et peut être identique à ou différent de n ou de o, n pouvant être un nombre de 2 à 6 et o un nombre entier de 0 à 4,
X peut avoir comme signification outre celle donnée pour R₅ : un acide aminé lié par une liaison amidique, un peptide, respectivement polypeptide lié par une liaison amidique, un groupe -C(O)-CHR₆N(R₇)₂, un groupe -C(O)-CHR₆N⁺(R₇)₃, un groupe -C(O)-CHR₆N⁺(R₇)₂R₈ ou un groupe -C(O)-CHR₆NR₇R₈, où
R₆ peut représenter un résidu -(CH₂)ₘ-NR₇R₈, un résidu -(CH₂)ₘ-N⁺(R₇)₃ ou un résidu -(CH₂)ₘ-N⁺(R₇)₂R₈ et m peut représenter un nombre de 1 à 5,
R₇ représente un atome d'hydrogène ou un groupe alkyle avec 1 à 4 atomes de carbone,
R₈ peut représenter un groupe -(CH₂)ₙ-N(R₇)₂ ou un groupe -(CH₂)ₙ-N⁺(R₇)₃ où n est un nombre de 2 à 4 et R₇ peut présenter la signification indiquée ci-dessus et
Y est un anion pharmaceutiquement acceptable,
B₁ représente un résidu NH[C(O)-(CH₂)ₚ-NH]_{q}-Z où p représente un nombre de 1 à 6 et q un nombre de 0 à 2,
Z représente un acide aminé lié par une liaison amidique, un peptide, respectivement polypeptide lié par une liaison amidique, un groupe -C(O)-CHR₆N(R₇)₂, un groupe -C(O)-CHR₆N⁺(R₇)₃, un groupe -C(O)-CHR₆N⁺(R₇)₂R₈ ou un groupe -C(O)-CHR₆NR₇R₈ et R₆ à R₈ et m présentent les significations indiquées ci-dessus,
et
B₂ représente un groupe O-R₂, où R₂ présente la signification indiquée pour R₁ et peut être identique à ou différent de R₁,
sous condition que la signification de A₁ ne vaut qu'avec B₁ et celle de A₂ qu'avec B₂.

8. Réactif selon la revendication 7, caractérisé en ce que la solution contient un mélange constitué d'au moins un composé selon la Formule générale (I) et d'un autre composé lipidique dans des solvants miscibles à l'eau.

9. Réactif selon la revendication 7 ou 8, caractérisé en ce qu'il s'agit dans le cas du solvant miscible à l'eau d'un alcool inférieur.

10. Réactif caractérisé en ce qu'un ou plusieurs composés des revendications 1 à 6, éventuellement en présence d'autres composés lipidiques, se trouvent sous forme de liposomes ou d'autres agrégats.

11. Procédé pour l'introduction de biomolécules dans des cellules, caractérisé en ce qu'on utilise un réactif selon les revendications 7 à 10 et en ce que celui-ci est d'abord mis en contact avec une biomolécule anionique et ensuite avec des cellules en vue de la transfection.

12. Procédé selon la revendication 11, caractérisé en ce qu'il s'agit dans le cas des molécules à introduire d'ADN ou d'ARN.

13. Procédé selon la revendication 11, caractérisé en ce que la biomolécule anionique est de l'ADN ou un fragment correspondant.

14. Procédé selon la revendication 11 à 13, caractérisé en ce qu'on utilise le 2-(6-carboxyspermyl)-1,3-dioléoyloxypropylamide, le 1-(6-carboxy-spermyl)-2,3-dioléoyloxypropylamide, le 2,3-dioléoyloxy-N-(N-(6-carboxy-spermyl)glycyl)aminopropane, le 2-(6-carboxyspermyl)-1,3-dimyristoyloxy-propylamide, le 2-(1,1,1,5,5,10,10,14,14,14-décaméthyl-6-carboxyspermyl)-1,3-dioléoyloxypropylamide, le 2-(N,N,N,N',N',N'-hexaméthylornithyl)-1,3-dioléoyl-oxypropylamide et/ou le 2-(N,N,N,N',N',N'-hexaméthyllysyl)-1,3-dioléoyloxy-propylamide, seuls ou éventuellement en présence d'un ou de plusieurs autres composés lipidiques.
